(19) **Europäisches Patentamt**

**European Patent Office**

**Office européen des brevets**

(11) Veröffentlichungsnummer : **0 464 517 A2**

(12) **EUROPÄISCHE PATENTANMELDUNG**

(21) Anmeldenummer : **91110259.8**

(22) Anmeldetag : **21.06.91**

(51) Int. Cl.⁵ : **C07K 5/02,** A61K 37/64,
C07D 233/64, C07D 401/12,
C07D 211/58, C07C 323/52,
A61K 31/195, A61K 31/415,
A61K 31/445

(30) Priorität : **05.07.90 DE 4021512**

(43) Veröffentlichungstag der Anmeldung :
**08.01.92 Patentblatt 92/02**

(84) Benannte Vertragsstaaten :
**AT BE CH DE DK ES FR GB GR IT LI LU NL SE**

(71) Anmelder : **MERCK PATENT GESELLSCHAFT
MIT BESCHRÄNKTER HAFTUNG
Frankfurter Strasse 250 Postfach 4119
W-6100 Darmstadt (DE)**

(72) Erfinder : **Raddatz, Peter, Dr.
Akazienweg 8A
W-6104 Seeheim (DE)**
Erfinder : **Minck, Klaus-Otto, Dr.
Büchestrasse 8
W-6105 Ober-Ramstadt (DE)**
Erfinder : **Schmitges, Claus J., Dr.
Karolingerstrasse 5
W-6114 Gross-Umstadt (DE)**

(54) **Aminosäurederivate mit renininhibierender Aktivität.**

㊦ Neue Aminosäurederivate der Formel I
$$X\text{-}W\text{-}CR^1R^2\text{-}CO\text{-}Y\text{-}NH\text{-}CHR^4\text{-}CR^5\text{-}CH_2\text{-}(CR^6R^7)_r S(O)_t\text{-}CH_2\text{-}CO\text{-}V\text{-}R^3 \qquad I$$
worin X, W, $R^1, R^2, Y, R^4, R^5, R^6, R^7, r, t, V$ und $R^3$ die in Patentanspruch 1 angegebenen Bedeutungen haben, hemmen die Aktivität des menschlichen Plasmarenins und können zur Behandlung von Herz-, Kreislauf- und Gefäßkrankheiten verwendet werden.

**EP 0 464 517 A2**

Die Erfindung betrifft neue Aminosäurederivate der der Formel I

$$\text{X-W-CR}^1\text{R}^2\text{-CO-Y-NH-CHR}^4\text{-CR}^5\text{-CH}_2\text{-(CR}^6\text{R}^7)_r\text{S(O)}_t\text{-CH}_2\text{CO-V-R}^3 \qquad \text{I}$$

worin

X  $R^8$, $R^8$ -O-$C_mH_{2m}$-CO-, $R^8$-$C_mH_{2m}$-O-CO-, $R_8$ -$C_mH_{2m}$-CO-, $R^8$-$SO_2$-, $R^9R^{10}N$-$C_mH_{2m}$-CO-, $R^{11}$-NH-C (=NH) -NH-$C_mH_{2m}$-CO-,$R^9$OOC-$C_mH_{2m}$-CO-, R $^9O_3$S-$C_mH_{2m}$-CO-, $R^9$-O- $(CH_2CH_2O)_n$-$C_mH_{2m}$-CO-, oder $A_3$N+-$C_mH_{2m}$-CO- An-,

W  O, NH, $CH_2$ oder S,

Y  0 oder 1 Aminosäurerest ausgewählt aus der Gruppe bestehend aus Abu, Ada, Ala, βAla, Arg, Asn, Asp, Bia, Cal, Cys, S-A-Cys, Dab, Gln, Glu, Gly, His, N(im)-A-His, Hph, Ile, Leu, tert.-Leu, Lys, Mal, Met, αNal, βNal, Nbg, Nle, Nva, Orn, Phe, Pia, Pro, Pya, Ser, Thr, Tia, Tic, Tiz, Trp, Tyr und Val,

V  O oder NH,

$R^1$, $R^6$, $R^7$,

$R^9$ und $R^{10}$  jeweils H oder A,

$R^2$, $R^3$, $R^4$ und $R^8$  jeweils H, A, Ar, Ar-alkyl, Het, Het-alkyl, unsubstituiertes oder ein- oder mehrfach durch A, AO und/oder Hal substituiertes Cycloalkyl mit 3-7 C-Atomen, Cycloalkyl-alkyl mit 4-11 C-Atomen, Bicycloalkyl oder Tricycloalkyl mit jeweils 7-14 C-Atomen, Bicycloalkylalkyl oder Tricycloalkylalkyl mit jeweils 8-18 C-Atomen,

$R^5$  (H, OH), (H, $NH_2$) oder =O,

$R^9R^{10}N$  auch eine unsubstituierte oder eine durch A, OH, $NH_2$, NHA, $NA_2$, NHAc, NH-CO-$C_xH_{2x}$-O-$R^{11}$, NH-CO-O-$C_xH_{2x}$-$R^{11}$, Hydroxy-alkyl, COOH, COOA, $CONH_2$, Amino-alkyl, HAN-alkyl, $A_2$N-alkyl, $A_3$N$^\oplus$al-kyl An$^\ominus$, NH-CO-$NH_2$, NH-CO-NHA, Guanidinyl oder Guanidinyl-alkyl substituierte Pyrrolidino-, Piperidino-, Morpholino- oder Piperazino-gruppe,

$R^{11}$  A oder Ar-alkyl,

m und x  jeweils 0, 1, 2, 3, 4, 5, 6, 7, 8, 9 oder 10,

n  1, 2, 3, 4 oder 5,

r  0, 1, 2 oder 3,

t  0, 1 oder 2,

Ar  unsubstituiertes oder ein- oder mehrfach durch A, OA, Hal, $CF_3$, OH, $NO_2$, Hydroxy-alkyl, $NH_2$, NHA, $NA_2$, NHAc, NH-$SO_2$-A, SA, SO-A, $SO_2$-A, $SO_2NH_2$, $SO_2$NHA, COOH, COOA, $CONH_2$, CN, Amino-alkyl, HAN-alkyl, $A_2$N-alkyl, $A_3$N$^\oplus$-alkyl An$^\ominus$ und/oder Guanidinyl-alkyl substituiertes Phenyl oder unsubstituiertes Naphthyl,

Het  einen gesättigten oder ungesättigten 5-oder 6-gliedrigen heterocyclischen Rest mit 1-4 N-, O-und/oder S-Atomen, der mit einem Benzolring kondensiert und/oder ein-oder mehrfach durch A, OA, Hal, $CF_3$, OH, $NO_2$, Carbonylsauerstoff, $NH_2$, NHA, $NA_2$, NHAc, NH-COOA, NHCOOAr, NHCOO$CH_2$Ar, NH-$SO_2$-A, SA, SO-A, $SO_2$-A, $SO_2NH_2$, $SO_2$NHA, COOH, COOA, $CONH_2$, CN, Ar, Ar-alkyl, Ar-alkenyl, Hydroxy-alkyl, Amino-alkyl, HAN-alkyl, $A_2$N-alkyl und/oder $A_3$N$^\oplus$-alkyl An$^\ominus$ substituiert sein kann und/oder dessen N- und/oder S-Heteroatome auch oxydiert sein können,

Hal  F, Cl, Br oder J,

Ac  A-CO-, Ar-CO-, Ar-alkyl-CO- oder A-NH-CO-,

An$^\ominus$  ein Anion, das auch fehlen kann, wenn stattdessen eine in der Verbindung der Formel I enthaltene Carboxygruppe in Form eines Carboxylatanions vorliegt,

-alkyl  eine Alkylengruppe mit 1-8 C-Atomen und

A  Alkyl mit 1-8 C-Atomen bedeuten,

worin ferner an Stelle einer oder mehrerer -CO-NH-Gruppen auch eine oder mehrere -CO-NA-Gruppen stehen können,

sowie deren Salze.

Ähnliche Verbindungen sind aus der EP-A-249096 bekannt.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze sehr wertvolle Eigenschaften besitzen. Vor allem hemmen sie die Aktivität des menschlichen Plasmarenins. Diese Wirkung kann z.B. nach der Methode von F. Fyhrquist et al., Clin.Chem. 22, 250-256 (1976), nachgewiesen werden. Bemerkenswert ist, daß diese Verbindungen sehr spezifische Hemmer des Renins sind; für die Hemmung anderer Aspartylproteinasen (z.B. Pepsin und Kathepsin D) sind in der Regel etwa 100 bis 1000mal so hohe Konzentrationen dieser Verbindungen notwendig wie für die Renin-Hemmung. Die Wirkungen der Verbindungen auf den Blutdruck und/oder auf die Herzfrequenz sowie die Hemmung der Reninaktivität im Blutplasma können ferner an wachen Affen, z.B. weiblichen Affen (Macaca fascicularis) ermittelt werden; dabei können Blutdruck und Herzfrequenz in Anlehnung an die Methode von M.J. Wood et al., J. Hypertension 4, 251-254 (1985) gemessen werden. Zur Stimulierung der Reninaktivität werden die Tiere dabei zweckmäßig mit einem Saluretikum vorbehandelt. Blutproben zur Bestimmung der Plasma-Reninaktivität können durch Punktion der Vena femoralis gewonnen werden.

Die Verbindungen können als Arzneimittelwirkstoffe in der Human- und Veterinärmedizin eingesetzt werden, insbesondere zur Prophylaxe und zur Behandlung von Herz-, Kreislauf- und Gefäßkrankheiten, vor allem von Hypertonie, Herzinsuffizienz und Hyperaldosteronismus. Außerdem können die Verbindungen zu diagnostischen Zwecken verwendet werden, um bei Patienten mit Hypertonie oder Hyperaldosteronismus den möglichen Beitrag der Reninaktivität zur Aufrechterhaltung des pathologischen Zustands zu bestimmen. Solche diagnostische Tests können ausgeführt werden ähnlich wie es in der EP-A-77 028 angegeben ist.

Die vor- und nachstehend aufgeführten Abkürzungen von Aminosäureresten stehen für die Reste -NR′-R″-CO-, in der Regel -NH-CHR-CO- (worin R, R′ und R″ die für jede Aminosäure bekannte spezifische Bedeutung haben) folgender Aminosäuren:

| | |
|---|---|
| Abu | 2-Aminobuttersäure |
| Ada | 3-(1-Adamantyl)-alanin |
| Ala | Alanin |
| βAla | β-Alanin |
| Arg | Arginin |
| Asn | Asparagin |
| Asp | Asparaginsäure |
| Bia | 3-(2-Benzimidazolyl)-alanin |
| Cal | 3-Cyclohexylalanin |
| Cys | Cystein |
| S-A-Cys | S-Alkyl-cystein |
| S-Me-Cys | S-Methyl-cystein |
| Dab | 2,4-Diaminobuttersäure |
| Gln | Glutamin |
| Glu | Glutaminsäure |
| Gly | Glycin |
| His | Histidin |
| N(im)-A-His | in 1- oder 3-Stellung des Imidazolrings durch A substituiertes Histidin |
| Hph | Homophenylalanin (2-Amino-4-phenyl-buttersäure) |
| Ile | Isoleucin |
| Leu | Leucin |
| tert.-Leu | tert.-Leucin |
| Lys | Lysin |
| Mal | 3-(p-Methoxyphenyl)-alanin |
| Met | Methionin |
| αNal | 3-(α-Naphthyl)-alanin |
| βNal | 3-(β-Naphthyl)-alanin |
| Nbg | 2-Norbornyl-glycin |
| Nle | Norleucin |
| N-Me-His | N-Methyl-histidin |
| N-Me-Phe | N-Methyl-phenylalanin |
| Nva | Norvalin |
| Orn | Ornithin |
| Phe | Phenylalanin |
| Pia | 3-(Piperidyl)-alanin [z.B. 2-Pia = 3-(2-Piperidyl)-alanin] |
| Pro | Prolin |
| Pya | 3-(Pyridyl)-alanin [z.B. 3-Pya = 3-(3-Pyridyl)-alanin] |
| Ser | Serin |
| Thr | Threonin |
| Tia | 3-(Thienyl)-alanin [z.B. 2-Tia = 3-(2-Thienyl)-alanin] |
| Tic | 1,2,3,4-Tetrahydroisochinolin-1-carbonsäure |
| Tiz | 3-(Thiazolyl)-alanin [z.B. 2-Tiz = 3-(2-Thiazolyl)-alanin] |
| Trp | Tryptophan |
| Tyr | Tyrosin |
| Val | Valin. |

Ferner bedeutet nachstehend:

BOC       tert.-Butoxycarbonyl
BOM      Benzyloxymethyl
imi-BOM  Benzyloxymethyl in 1-Stellung des Imidazolrings
CBZ      Benzyloxycarbonyl
DCCI     Dicyclohexylcarbodiimid
DMF     Dimethylformamid
DNP     2,4-Dinitrophenyl
imi-DNP  2,4-Dinitrophenyl in 1-Stellung des Imidazolrings
ETOC    Ethoxycarbonyl
FMOC   9-Fluorenylmethoxycarbonyl
HOBt    1-Hydroxybenzotriazol
IPOC    Isopropoxycarbonyl
Pla       den Rest der Phenylmilchsäure -O-CH(CH$_2$C$_6$H$_5$)-CO- (S-Form)
POA     Phenoxyacetyl
THF     Tetrahydrofuran.

Sofern die vorstehend genannten Aminosäuren in mehreren enantiomeren Formen auftreten können, so sind vor- und nachstehend, z.B. als Bestandteil der Verbindungen der Formel I, alle diese Formen und auch ihre Gemische (z.B. die DL-Formen) eingeschlossen. Die L-Formen sind bevorzugt. Sofern nachstehend einzelne Verbindungen aufgeführt sind, so beziehen sich die Abkürzungen dieser Aminosäuren jeweils auf die L-Form, sofern nicht ausdrücklich etwas anderes angegeben ist.

Gegenstand der Erfindung ist ferner ein Verfahren zur Herstellung eines Aminosäurederivats der Formel I sowie von seinen Salzen, dadurch gekennzeichnet, daß man es aus einem seiner funktionellen Derivate durch Behandeln mit einem solvolysierenden oder hydrogenolysierenden Mittel in Freiheit setzt oder daß man eine Carbonsäure der Formel II

$$X\text{-}G^1\text{-}OH \qquad II$$

worin G$^1$

(a)      fehlt,
(b)      -W-CR$^1$R$^2$-CO-,
(c)      -W-CR$^1$R$^2$-CO-Y-,
(d)      -W-CR$^1$R$^2$-CO-Y-NH-CHR$^4$-CR$^5$-CH$_2$-(CR$^6$R$^7$)$_r$-S(O)$_t$-CH$_2$-CO- bedeutet,

oder eines ihrer reaktionsfähigen Derivate
mit einer Verbindung der Formel III

$$H\text{-}G^2\text{-}V\text{-}R^3 \qquad III$$

worin G$^2$

(a)      -W'-CR$^1$R$^2$-CO-Y-NH-CHR$^4$-CR$^5$-CH$_2$ (CR$^6$R$^7$)$_r$-S(O)$_t$-CH$_2$-CO-,
(b)      -Y-NH-CHR$^4$-CR$^5$-CH$_2$-(CR$^6$R$^7$)$_r$-S(O)$_t$-CH$_2$-CO- bedeutet,
(c)      -NH-CHR$^4$-CR$^5$-CH$_2$-(CR$^6$R$^7$)$_r$-S(O)$_t$-CH$_2$-CO-bedeutet,
(d)      fehlt, und
     W' O, NH oder S bedeutet,

umsetzt,
und daß man gegebenenfalls in einer Verbindung der Formel I eine funktionell abgewandelte Amino- und/ oder Hydroxygruppe durch Behandeln mit solvolysierenden oder hydrogenolysierenden Mitteln in Freiheit setzt und/oder eine freie Aminogruppe durch Behandeln mit einem acylierenden Mittel acyliert und/oder zur Herstellung einer Verbindung der Formel I, R$^5$ = (H, OH) oder (H, NH$_2$), ein Aminoketosäurederivat der Formel I, R$^5$ = O, reduziert oder reduktiv aminiert und/oder eine Thioethergruppe zu einer Sulfoxid- oder Sulfongruppe oxydiert und/oder eine Verbindung der Formel I durch Behandeln mit einer Säure in eines ihrer Salze überführt.

Vor- und nachstehend haben die Reste bzw. Parameter R$^1$ bis R$^{11}$, V, W, X, Y, m, n, x, r, t, Ar, Het, Hal, Ac, An, A, G$^1$, G$^2$ und W' die bei den Formeln I, II oder III angegebenen Bedeutungen, falls nicht ausdrücklich etwas anderes angegeben ist.

In den vorstehenden Formeln hat A 1-8, vorzugsweise 1, 2, 3 oder 4 C-Atome. A bedeutet vorzugsweise Methyl, weiterhin Ethyl, Propyl, Isopropyl, Butyl, Isobutyl, sek.-Butyl, oder tert.-Butyl, ferner auch Pentyl, 1-, 2- oder 3-Methylbutyl, 1,1-, 1,2- oder 2,2-Dimethylpropyl, 1-Ethylpropyl, Hexyl, 1-, 2-, 3- oder 4-Methylpentyl, 1,1-, 1,2-, 1,3-, 2,2-, 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl, Heptyl, Octyl.

Cycloalkyl bedeutet vorzugsweise Cyclopropyl, Cyclobutyl, Cyclopentyl, Cyclohexyl oder Cycloheptyl, aber auch z.B. 1-, 2- oder 3-Methylcyclopentyl, 1-, 2-, 3- oder 4-Methylcyclohexyl.

Dementsprechend bedeutet Cycloalkylalkyl vorzugsweise Cyclopropylmethyl, 2-Cyclopropylethyl, Cyclobutylmethyl, 2-Cyclobutylethyl, Cyclopentylmethyl, 2-Cyclopentylethyl, Cyclohexylmethyl, 2-Cyclohexylethyl, aber auch z.B. 1-, 2- oder 3-Methylcyclopentylmethyl, 1-, 2-, 3- oder 4-Methylcyclohexylmethyl.

Bicycloalkyl bedeutet vorzugsweise 1- oder 2-Dekalyl, 2-Bicyclo[2,2,1]heptyl oder 6,6-Dimethyl-2-bicyclo-[3,1,1]heptyl.

Tricycloalkyl bedeutet vorzugsweise 1-Adamantyl.

Hal bedeutet vorzugsweise F, Cl oder Br, aber auch J.

Ac bedeutet vorzugsweise A-CO-, wie Acetyl, Propionyl oder Butyryl, Ar-CO- wie Benzoyl, o-, m- oder p-Methoxybenzoyl oder 3,4-Dimethoxybenzoyl, A-NH-CO- wie N-Methyl-oder N-Ethylcarbamoyl.

Ar bedeutet vorzugsweise Phenyl, ferner bevorzugt o-, m- oder p-Tolyl, o-, m- oder p-Ethylphenyl, o-, m- oder p-Methoxyphenyl, o-, m- oder p-Fluorphenyl, o-, m- oder p-Chlorphenyl, o-, m- oder p-Bromphenyl, o-, m- oder p-Jodphenyl, o-, m- oder p-Trifluormethylphenyl, o-, m-oder p-Hydroxyphenyl, o-, m- oder p-Sulfamoylphenyl, 2,3-, 2,4-, 2,5-, 2,6-, 3,4- oder 3,5-Dimethoxyphenyl, 3,4,5-Trimethoxyphenyl, o-, m- oder p-Aminophenyl, o-, m- oder p-Aminomethylphenyl, o-, m- oder p-Dimethylaminomethylphenyl, o-, m- oder p-Guanidinomethylphenyl, 1- oder 2-Naphthyl.

Dementsprechend bedeutet Ar-alkyl vorzugsweise Benzyl, 1-oder 2-Phenylethyl, o-, m- oder p-Methylbenzyl, 1-oder 2-o-, -m- oder -p-Tolylethyl, o-, m- oder p-Ethylbenzyl, 1- oder 2-o-, -m- oder -p-Ethylphenylethyl, o-, m- oder p-Methoxybenzyl, 1- oder 2-o-, -m- oder -p-Methoxyphenylethyl, o-, m- oder p-Fluorbenzyl, 1- oder 2-o-, -m- oder -p-Fluorphenylethyl, o-, m- oder p-Chlorbenzyl, 1- oder 2-o-, -m- oder -p-Chlorphenylethyl, o-, m- oder p-Brombenzyl, 1- oder 2-o-, -m- oder -p-Bromphenylethyl, o-, m- oder p-Jodbenzyl, 1- oder 2-o-, -m- oder -p-Jodphenylethyl, o-, m- oder p-Trifluormethylbenzyl, o-, m- oder p-Hydroxybenzyl, 2,3-, 2,4-, 2,5-, 2,6-, 3,4- oder 3,5-Dimethoxybenzyl, 3,4,5-Trimethoxybenzyl, o-, m- oder p-Aminobenzyl, o-, m- oder p-Aminomethylbenzyl, o-, m- oder p-Dimethylaminomethylbenzyl, o-, m- oder p-Guanidinomethylbenzyl, 1- oder 2-Naphthylmethyl.

Het ist vorzugsweise 2- oder 3-Furyl, 2- oder 3-Thienyl, 1-, 2- oder 3-Pyrrolyl, 1-, 2-, 4- oder 5-Imidazolyl, 1-, 3-, 4-oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4-oder -5-yl, 1,2,4-Triazol-1-, -3- oder -5-yl, 1- oder 5-Tetrazolyl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3- oder -5-yl, 2,1,5-Thiadiazol-3- oder -4-yl, 2-, 3-, 4-, 5- oder 6-2H-Thiopyranyl, 2-, 3- oder 4-4H-Thiopyranyl, 3- oder 4-Pyridazinyl, Pyrazinyl, 2-, 3-, 4-, 5-, 6- oder 7-Benzofuryl, 2-, 3-, 4-, 5-, 6- oder 7-Benzothienyl, 1-, 2-, 3-, 4-, 5-, 6- oder 7-Indolyl, 1-, 2-, 3-, 4-, 5-, 6- oder 7-Isoindolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7-Benzisoxazolyl, 2-, 4-, 5-, 6- oder 7-Benzthiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-Isochinolyl, 1-, 2-, 3-, 4- oder 9-Carbazolyl, 1-, 2-, 3-, 4-, 5-, 6-, 7-, 8- oder 9-Acridinyl, 3-, 4-, 5-, 6-, 7- oder 8-Cinnolyl, 2-, 4-, 5-, 6- 7- oder 8-Chinazolyl. Die heterocyclischen Reste können auch teilweise oder vollständig hydriert sein. Het kann also z.B. auch bedeuten 2,3-Dihydro-2-, -3-, -4- oder -5-furyl, 2,5-Dihydro-2-, -3-, -4- oder -5-furyl, Tetrahydro-2- oder -3-furyl, Tetrahydro-2- oder -3-thienyl, 2,3-Dihydro-1-, -2-, -3-, -4- oder -5-pyrrolyl, 2,5-Dihydro-1-, -2-, -3-, -4- oder -5-pyrrolyl, 1-, 2- oder 3-Pyrrolidinyl, Tetrahydro-1-, -2- oder -4-imidazolyl, 2,3-Dihydro-1-, -2-, -3-, -4- oder -5-pyrazolyl, Tetrahydro-1-, -3- oder -4-pyrazolyl, 1,4-Dihydro-1-, -2-, -3-oder -4-pyridyl, 1,2,3,4-Tetrahydro-1-, -2-, -3-, -4-, -5- oder -6-pyridyl, 1,2,3,6-Tetrahydro-1-, -2-, -3-, -4-, -5- oder -6-pyridyl, 1-, 2-, 3- oder 4-Piperidinyl, 2-, 3- oder 4-Morpholinyl, Tetrahydro-2-, -3- oder -4-pyranyl, 1,4-Dioxanyl, 1,3-Dioxan-2-, -4- oder -5-yl, Hexahydro-1-, -3- oder -4-pyridazinyl, Hexahydro-1-, -2-, -4- oder -5-pyrimidinyl, 1-, 2- oder 3-Piperazinyl, 1,2,3,4-Tetrahydro-1-, -2-, -3-, -4-, -5-, -6-, -7- oder -8-chinolyl, 1,2,3,4-Tetrahydro-1-, -2-, -3-, -4-, -5-, -6-, -7- oder 8-isochinolyl.

Die heterocyclischen Reste können auch wie angegeben substituiert sein. Het kann z.B. bevorzugt auch bedeuten: 2-Amino-4-thiazolyl, 4-Carboxy-2-thiazolyl, 4-Carbamoyl-2-thiazolyl, 4-(2-Aminoethyl)-2-thiazolyl, 4-Amino-2-methyl-5-pyrimidinyl, 2-Amino-5,6-dimethyl-3-pyrazinyl, 4-Carbamoylpiperidino, ferner z.B. 3-, 4- oder 5-Methyl-2-furyl, 2-, 4- oder 5-Methyl-3-furyl, 2,4-Dimethyl-3-furyl, 5-Nitro-2-furyl, 5-Styryl-2-furyl, 3-, 4- oder 5-Methyl-2-thienyl, 2-, 4- oder 5-Methyl-3-thienyl, 3-Methyl-5-tert.-butyl-2-thienyl, 5-Chlor-2-thienyl, 5-Phenyl-2- oder -3-thienyl, 1-, 3-, 4- oder 5-Methyl-2-pyrrolyl, 1-Methyl-4- oder -5-nitro-2-pyrrolyl, 3,5-Dimethyl-4-ethyl-2-pyrrolyl, 4-Methyl-5-pyrazolyl, 5-Methyl-3-isoxazolyl, 3,4-Dimethyl-5-isoxazolyl, 4- oder 5-Methyl-2-thiazolyl, 2- oder 5-Methyl-4-thiazolyl, 2- oder 4-Methyl-5-thiazolyl, 2,4-Dimethyl-5-thiazolyl, 3-, 4-, 5- oder 6-Methyl-2-pyridyl, 2-, 4-, 5- oder 6-Methyl-3-pyridyl, 2- oder 3-Methyl-4-pyridyl, 3-, 4-, 5- oder 6-Chlor-2-pyridyl, 2-, 4-, 5- oder 6-Chlor-3-pyridyl, 2- oder 3-Chlor-4-pyridyl, 2,6-Dichlorpyridyl, 2-Hydroxy-3-, -4-, -5- oder -6-pyridyl (= 1H-2-Pyridon-3-, -4-, -5- oder -6-yl), 5-Phenyl-1H-2-pyridon-3-yl, 5-p-Methoxyphenyl-1H-2-pyridon-3-yl, 2-Methyl-3-hydroxy-4-hydroxymethyl-5-pyridyl, 2-Hydroxy-4-amino-6-methyl-3-pyridyl, 3-N'-Methylureido-1H-4- pyridon-5-yl, 4-Methyl-2-pyrimidinyl, 4,6-Dimethyl-2-pyrimidinyl, 2-, 5- oder 6-Methyl-4-pyrimidinyl, 2,6-Dimethyl-4- pyrimidinyl, 2,6-Dihydroxy-4-pyrimidinyl, 5-Chlor-2-methyl-4- pyrimidinyl, 3-Methyl-2-benzofuryl, 2-Ethyl-3-benzofuryl, 7-Methyl-2-benzothienyl, 1-, 2-, 4-, 5-, 6-oder 7-Methyl-3- indolyl, 1-Methyl-5- oder -6-ben-

5

zimidazolyl, 1-Ethyl-5- oder -6-benzimidazolyl, 3-, 4-, 5-, 6-, 7- oder 8-Hydroxy-2-chinolyl, 2-Oxo-pyrrolidino, 2-Oxo-piperidino, 2,5-Dioxopyrrolidino, 3-Benzyl-2,5-dioxopyrrolidino. X ist allgemein vorzugsweise $R^8$, $R^8$-$C_mH_{2m}$-O-CO-, $R^9R^{10}$N-$C_mH_{2m}$-CO-, insbesondere 4-BOC-amino-piperidinocarbonyl, 4-Amino-piperidinocarbonyl, 3-BOC-amino-3-methyl-butyryl oder 3-Amino-3-methyl-butyryl, oder $R^8$-$SO_2$-, insbesondere A-$SO_2$- wie tert.-Butyl-sulfonyl oder Isopropyl-sulfonyl.

Die Gruppe Y besteht vorzugsweise aus einem der angegebenen Aminosäurereste; sie kann jedoch auch fehlen. Vorzugsweise bedeutet Y βAla, His, S-Me-Cys oder Nva, ferner bevorzugt Asn, Dab, Gln, Gly, N-Me-His, Ile, Leu, tert.-Leu, Lys, Met, Nle, Orn, Pya (besonders 3-Pya), Ser, Tia oder Val.

V ist vorzugsweise NH.

$R^1$, $R^6$, $R^7$, $R^9$ und $R^{10}$ bedeuten jeweils bevorzug H, ferner bevorzugt Methyl; $R^6$ ist bevorzugt auch Isopropyl oder Isobutyl. $R^9R^{10}$N ist bevorzugt auch Pyrrolidino, Piperidino, Morpholino, Amino-piperidino wie 4-Aminopiperidino, Alkylaminopiperidino wie 4-Methylaminopiperidino, Dialkylaminopiperidino wie 4-Dimethylaminopiperidino oder BOC-amino-piperidino wie 4-BOC-amino-piperidino. $CR^6R^7$ ist vorzugsweise $CH_2$.

$R^2$ ist vorzugsweise Ar-alkyl, insbesondere Benzyl oder p-Methoxybenzyl; ferner bevorzugt A, insbesondere n-Butyl oder Isobutyl; Cycloalkyl-alkyl, insbesondere Cyclohexylmethyl; Het-alkyl, insbesondere 2-Thienylmethyl. Die Gruppe -W-$CR^1R^2$-CO- bedeutet bevorzugt einen der Reste Phe oder Pla, ferner Ada, Bia, Cal, Hph, Leu, Mal, Tia oder Trp.

$R^3$ ist vorzugsweise H oder A.

$R^4$ ist vorzugsweise Cycloalkylalkyl, insbesondere Cyclohexylmethyl, ferner bevorzugt Alkyl, insbesondere n-Butyl oder Isobutyl; Ar-alkyl, insbesondere Benzyl oder p-Methoxybenzyl; Het-alkyl, z. B. 2-Thienylmethyl; Cycloalkyl, insbesondere Cyclohexyl.

$R^5$ ist vorzugsweise (H, OH).

Die Gruppe -($CR^6R^7$)$_r$- ist vorzugsweise -$CH_2$- oder -$CH_2CH_2$-.

$R^{11}$ ist vorzugsweise H, Methyl oder CN.

Der Parameter m ist vorzugsweise 1, 2, 3, 4 oder 5; n ist vorzugsweise 1; r ist vorzugsweise 0 oder 1; t ist vorzugsweise 0 oder 2; x ist vorzugsweise 1 oder 2.

$C_mH_{2m}$ und $C_xH_{2x}$ sind vorzugsweise geradkettig, bedeuten also vorzugsweise -($CH_2$)$_m$- oder -($CH_2$)$_x$-.

Dementsprechend bedeutet die Gruppe X im einzelnen vorzugsweise A, z. B. Isopropyl oder Isobutyl; Ar, z.B. Phenyl; Ar-alkyl, z. B. Benzyl; $R^9R^{10}$N-($CH_2$)$_m$-CO-, vor allem $H_2$N-$C_mH_{2m}$-CO- wie Aminocarbonyl, Aminoacetyl (H-Gly-), 3-Aminopropionyl (H-βAla-), 4-Aminobutyryl, 5-Aminopentanoyl, 6-Aminohexanoyl, 7-Aminoheptanoyl, 8-Aminooctanoyl, 9-Aminononanoyl, 10-Aminodecanoyl, 11-Aminoundecanoyl, aber auch z.B. 2-Aminopropionyl(Ala), 2-Amino-2-methyl-propionyl, 3-Amino-3-methylbutyryl; ANH-$C_mH_{2m}$-CO- wie Methylaminocarbonyl, Methylaminoacetyl (Sarcosyl), 3-Methylaminopropionyl, 4-Methylaminobutyryl, 5-Methylaminopentanoyl, 6-Methylaminohexanoyl, 6-Ethylaminohexanoyl, 7-Methylaminoheptanoyl, 8-Methylaminooctanoyl, 9-Methylaminononanoyl, 10-Methylaminodecanoyl, 11-Methylaminoundecanoyl; $A_2$N-$C_mH_{2m}$-CO- wie Dimethylaminocarbonyl, Dimethylaminoacetyl, 3-Dimethylaminopropionyl, 4-Dimethylaminobutyryl, 5-Dimethylaminopentanoyl, 6-Dimethylaminohexanoyl, 6-Diethylaminohexanoyl, 7-Dimethylaminoheptanoyl, 8-Dimethylaminooctanoyl, 9-Dimethylaminononanoyl, 10-Dimethylaminodecanoyl, 11-Dimethylaminoundecanoyl, Pyrrolidino-$C_mH_{2m}$-CO- wie Pyrrolidinocarbonyl, Pyrrolidino-acetyl, 3-Pyrrolidino-propionyl, 4-Pyrrolidino-butyryl, 5-Pyrrolidino-pentanoyl, 6-Pyrrolidino-hexanoyl, 7-Pyrrolidino-heptanoyl, 8-Pyrrolidino-octanoyl, 9-Pyrrolidino-nonanoyl, 10-Pyrrolidino-decanoyl; Piperidino-$C_mH_{2m}$-CO- wie Piperidinocarbonyl, Piperidinoacetyl, 3-Piperidino-propionyl, 4-Piperidino-butyryl, 5-Piperidino-pentanoyl, 6-Piperidino-hexanoyl, 7-Piperidinoheptanoyl, 8-Piperidino-octanoyl, 9-Piperidino-nonanoyl, 10-Piperidino-decanoyl; Morpholino-$C_mH_{2m}$-CO- wie Morpholinocarbonyl, Morpholinoacetyl, 3-Morpholino-propionyl, 4-Morpholino-butyryl, 5-Morpholino-pentanoyl, 6-Morpholino-hexanoyl, 7-Morpholino-heptanoyl, 8-Morpholino-octanoyl, 9-Morpholino-nonanoyl, 10-Morpholino-decanoyl; 4-Amino-piperidino- $C_mH_{2m}$-CO- wie 4-Amino-piperidinocarbonyl, 4-Amino-piperidino-acetyl, 3-(4-Aminopiperidino)-propionyl, 4-(4-Amino-piperidino)-butyryl, 5-(4-Amino-piperidino)-pentanoyl, 6-(4-Amino-piperidino)-hexanoyl, 7-(4-Amino-piperidino)-heptanoyl, 8-(4-Amino-piperidino)-octanoyl, 9-(4-Amino-piperidino)-nonanoyl, 10-(4-Amino-piperidino)-decanoyl; 4-BOC-amino-piperidino-$C_mH_{2m}$-CO- wie 4-BOC-amino-piperidino-carbonyl, 4-BOC-amino-piperidino-acetyl; 4-Dialkylamino-piperidino-$C_mH_{2m}$-CO-wie 4-Dimethylamino-piperidinocarbonyl, 4-Dimethylamino-piperidino-acetyl; 4-Guanidino-piperidino-$C_mH_{2m}$-CO- wie 4-Guanidino-piperidino-carbonyl, 4-Guanidino-piperidinoacetyl; 4-Carboxy-piperidino-$C_mH_{2m}$-CO- wie 4-Carboxy-piperidino-carbonyl, 4-Carboxy-piperidino-acetyl; 4-Alkoxycarbonyl-piperidino- $C_mH_{2m}$-CO- wie 4-Methoxycarbonyl-piperidino-carbonyl, 4-Ethoxycarbonyl-piperidino-carbonyl, 4-Methoxycarbonyl-piperidino-acetyl, 4-Ethoxycarbonyl-piperidino-acetyl; 4-AcNH-piperidino-$C_mH_{2m}$-CO- wie 4-Acetamido-piperidino-carbonyl, 4-Acetamido-piperidino-acetyl; $H_2$N-C(=NH)- NH-$C_mH_{2m}$-CO- wie Guanidinoacetyl, 3-Guanidino-propionyl, 4-Guanidino-butyryl, 5-Guanidino-pentanoyl, 6-Guanidino-hexanoyl, 7-Guanidino-heptanoyl, 8-Guanidino-octanoyl; NC-NH-C(=NH)-NH-$C_mH_{2m}$-CO- wie N'-Cyanguanidinoacetyl, 3-(N'-Cyanguanidino)-propionyl, 4-(N′-Cyanguanidino)-butyryl, 5-(N′-Cyanguanidino)-pentanoyl, 6-(N′-Cyanguanidino)-hexanoyl, 7-(N′-Cyanguanidino)-heptanoyl, 8-(N′-Cyanguanidino)-octanoyl; $HOOC-C_mH_{2m}-CO-$ wie Malonyl, Succinyl, Glutaryl, Adipyl, 6-Carboxyhexanoyl, 7-Carboxyheptanoyl, 8-Carboxyoctanoyl, 9-Carboxynonanoyl, 10-Carboxy-decanoyl, 11-Carboxyundecanoyl; $AOOC-C_mH_{2m}-CO-$ wie Methoxycarbonyl-acetyl, 3-Methoxycarbonyl-propionyl, 4-Methoxycarbonyl-butyryl, 5-Methoxycarbonyl-pentanoyl, 6-Methoxycarbonyl-hexanoyl, 7-Methoxycarbonyl-heptanoyl, 8-Methoxycarbonyl-octanoyl, 9-Methoxycarbonyl-nonanoyl, 10-Methoxycarbonyl-decanoyl, Ethoxycarbonyl-acetyl, 3-Ethoxycarbonyl-propionyl, 4-Ethoxycarbonyl-butyryl, 5-Ethoxycarbonyl-pentanoyl, 6-Ethoxycarbonyl-hexanoyl, 7-Ethoxycarbonyl-heptanoyl, 8-Ethoxycarbonyl-octanoyl, 9-Ethoxycarbonyl-nonanoyl, 10-Ethoxycarbonyl-decanoyl; $H-SO_3-C_mH_{2m}-CO-$ wie Sulfo-acetyl, 3-Sulfo-propionyl, 4-Sulfobutyryl, 5-Sulfo-pentanoyl, 6-Sulfo-hexanoyl, 7-Sulfoheptanoyl, 8-Sulfo-octanoyl, 9-Sulfo-nonanoyl, 10-Sulfodecanoyl; $A-SO_3-C_mH_{2m}-CO-$ wie Methoxysulfonyl-acetyl, 3-Methoxysulfonyl-propionyl, 4-Methoxysulfonyl-butyryl, 5-Methoxysulfonyl-pentanoyl, 6-Methoxysulfonyl-hexanoyl, 7-Methoxysulfonyl-heptanoyl, 8-Methoxysulfonyl-octanoyl, 9-Methoxysulfonyl-nonanoyl, 10-Methoxysulfonyl-decanoyl, Ethoxysulfonyl-acetyl, 3-Ethoxysulfonyl-propionyl, 4-Ethoxysulfonyl-butyryl, 5-Ethoxysulfonyl-pentanoyl, 6-Ethoxysulfonyl-hexanoyl, 7-Ethoxysulfonyl-heptanoyl, 8-Ethoxysulfonyl-octanoyl, 9-Ethoxysulfonyl-nonanoyl, 10-Ethoxysulfonyl-decanoyl;

$R^8-C_mH_{2m}-O-CO-$, vor allem A-O-CO- wie ETOC, IPOC, BOC sowie $Ar-C_mH_{2m}-O-CO-$ wie CBZ; $R^8-C_mH_{2m}-CO-$, vor allem A-CO- wie Acetyl, Trimethylacetyl oder 3,3-Dimethylbutyryl.

Die Verbindungen der Formel I können ein oder mehrere chirale Zentren besitzen und daher in verschiedenen - optisch-aktiven oder optisch-inaktiven - Formen vorkommen. Die Formel I umschließt alle diese Formen. Falls $R^4$ von H verschieden ist und/oder $R^5$ (H, OH) oder (H, $NH_2$) bedeutet, sind für r = 0 die 5S-Hydroxy-, 5S-Amino-, 6S-Amino-, 5S-Hydroxy-6S-amino- und 5S,6S-Diamino-Enantiomeren bzw. für r = 1 die 6S-Hydroxy-, 6S-Amino-, 7S-Amino-, 6S-Hydroxy-7S-amino und 6S,7S-Diamino-Enantiomeren bevorzugt (wobei dem C-Atom, das den Rest $R^5$ trägt, für r = 0 die 5-Stellung, für r = 1 die 6-Stellung, dem C-Atom, das die Reste $X-W-CR^1R^2-CO-Y-NR^3$ und $R^4$ trägt, für r = 0 die 6-Stellung, für r = 1 die 7-Stellung zugeordnet ist).

Die oben erwähnten Cycloalkyl- und Phenylgruppen sind vorzugsweise unsubstituiert oder tragen vorzugsweise 1 bis 3, insbesondere 1 oder 2 Substituenten.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln Ia bis Ik ausgedrückt werden:

Ia $\quad R^8-W-CR^1R^2-CO-Y-NH-CHR^4-CR^5-CH_2-(CR^6R^7)_r-S(O)_t-CH_2CO-V-R^3$;

Ib $\quad R^8-O-C_mH_{2m}-CO-W-CR^1R^2-CO-Y-NH-CHR^4-CR^5-CH_2-(CR^6R^7)_r-S(O)_t-CH_2CO-V-R^3$;

Ic $\quad R^8-C_mH_{2m}-O-CO-W-CR^1R^2-CO-Y-NH-CHR^4-CR^5-CH_2-(CR^6R^7)_r-S(O)_t-CH_2CO-V-R^3$;

Id $\quad R^8-C_mH_{2m}-CO-W-CR^1R^2-CO-Y-NH-CHR^4-CR^5-CH_2-(CR^6R^7)_r-S(O)_t-CH_2CO-V-R^3$;

Ie $\quad R^9R^{10}N-C_mH_{2m}-CO-W-CR^1R^2-CO-Y-NH-CHR^4-CR^5-CH_2-(CR^6R^7)_r-S(O)_t-CH_2CO-V-R^3$;

If $\quad R^{11}-NH-C(=NH)-NH-C_mH_{2m}-CO-W-CR^1R^2-CO-Y-NH-CHR^4-CR^5-CH_2-(CR^6R^7)_r-S(O)_t-CH_2CO-V-R^3$;

Ig $\quad R^9OOC-C_mH_{2m}-CO-W-CR^1R^2-CO-Y-NH-CHR^4-CR^5-CH_2-(CR^6R^7)_r-S(O)_t-CH_2CO-V-R^3$;

Ih $\quad R^9O_3S-C_mH_{2m}-CO-W-CR^1R^2-CO-Y-NH-CHR^4-CR^5-CH_2-(CR^6R^7)_r-S(O)_t-CH_2CO-V-R^3$ ;

Ii $\quad R^9-O-(CH_2CH_2O)_n-C_mH_{2m}-CO-W-CR^1R^2-CO-Y-NH-CHR^4-CR^5-CH_2-(CR^6R^7)_r-S(O)_t-CH_2CO-V-R^3$;

Ij $\quad R^9R^{10}N-CO-W-CR^1R^2-CO-Y-NH-CHR^4-CR^5-CH_2-(CR^6R^7)_r-S(O)_t-CH_2CO-V-R^3$;

Ik $\quad$ 4-Aminopiperidinocarbonyl-$W-CR^1R^2-CO-Y-NH-CHR^4-CR^5-CH_2-(CR^6R^7)_r-S(O)_t-CH_2CO-V-R^3$.

Insbesondere bevorzugt sind Verbindungen der Teilformeln:

(a) Iaa bis Ika, die den Formeln Ia bis Ik entsprechen, worin jedoch zusätzlich

$-W-CR^1R^2-CO-$ Phe, Pla, Mal oder $-CH_2-CH(CH_2C_6H_5)-CO-$bedeutet;

(b) Iab bis Ikb sowie Iaab bis Ikab, die den Formeln Ia bis Ik sowie Iaa bis Ika entsprechen, worin jedoch zusätzlich

Y $\quad$ βAla, His, Leu oder S-Me-Cys bedeutet.

(c) Iac bis Ikc, Iaac bis Ikac sowie Iabc bis Ikbc, die den Formeln Ia bis Ik, Iaa bis Ika sowie Iab bis Ikb entsprechen, worin jedoch zusätzlich $R^4$ Cyclohexylmethyl bedeutet.

Insbesondere sind bevorzugt Verbindungen der Teilformeln:

I* sowie Ia* bis Ik*, die den Formeln I sowie Ia bis Ik sowie solche Verbindungen, die den anderen vorstehend genannten Teilformeln entsprechen, worin jedoch zusätzlich

$R^5$ $\quad$ (H, OH) bedeutet;

I′ sowie Ia′ bis Ik′, die den Formeln I sowie Ia bis Ik sowie solche Verbindungen, die den anderen vorstehend genannten Teilformeln entsprechen, worin jedoch zusätzlich

$(CR^6R^7)_r$ $\quad$ $CH_2$ oder $CH_2CH_2$ bedeutet;

I″ sowie Ia″ bis Ik″, die den Formeln I sowie Ia bis Ik sowie solche Verbindungen, die den anderen vorstehend genannten Teilformeln entsprechen, worin jedoch zusätzlich

V       O oder NH und

$R^3$     H oder A bedeutet.

Eine besonders bevorzugte Gruppe von Verbindungen entspricht der Formel I,

worin

X      4-BOC-aminopiperidinocarbonyl, 4-Aminopiperidinocarbonyl, Morpholinocarbonyl oder A-SO$_2$-,

-W-CR$^1$R$^2$-CO-     Phe, Pla oder -CH$_2$-CH(CH$_2$C$_6$H$_5$)-CO-,

Y      βAla, His oder S-Me-Cys,

$R^3$     H oder A,

$R^4$     Cyclohexylmethyl,

$R^5$     (H, OH),

(CR$^6$R$^7$)$_r$     CH$_2$ oder CH$_2$CH$_2$,

V      O oder NH und

t      O oder 2 bedeuten.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart; ferner EP-A-45665, EP-A-77028, EP-A-77029, EP-A-81783, EP-A-249096) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

Die Verbindungen der Formel I können erhalten werden, indem man sie aus ihren funktionellen Derivaten durch Solvolyse, insbesondere Hydrolyse, oder durch Hydrogenolyse in freiheit setzt.

Bevorzugte Ausgangsstoffe für die Solvolyse bzw. Hydrogenolyse sind solche, die sonst der Formel I entsprechen, aber an Stelle einer oder mehrerer freier Amino- und/oder Hydroxygruppen entsprechende geschützte Amino- und/oder Hydroxygruppen enthalten, vorzugsweise solche, die an Stelle eines H-Atoms, das mit einem N-Atom verbunden ist, eine Aminoschutzgruppe tragen, z.B. solche die der Formel I entsprechen, aber an Stelle einer His-Gruppe eine N(im)-R'-His-Gruppe (worin R' eine Aminoschutzgruppe bedeutet, z.B. BOM oder DNP) enthalten, solche der Formel X-W-CR$^1$R$^2$-CO-Y-NH-CHR$^4$-CH(NHR')-CH$_2$-(CR$^6$R$^7$)$_r$-S(O)$_t$-CH$_2$CO-V-R$^3$ oder solche, die an Stelle einer Dab-, Lys- oder Orn-Gruppe eine entsprechende Gruppe enthalten, die an Stelle der endständigen NH$_2$-Gruppe eine NH-R'-Gruppe (z.B. NH-CBZ) enthält.

Ferner sind Ausgangsstoffe bevorzugt, die an Stelle des H-Atoms einer Hydroxygruppe eine Hydroxyschutzgruppe tragen, z.B. solche der Formel X-W-CR$^1$R$^2$-CO-Y-NH-CHR$^4$-CHOR''-CH$_2$-(CR$^6$R$^7$)$_r$-S(O)$_t$-CH$_2$CO-V-R$^3$, worin R'' eine Hydroxyschutzgruppe bedeutet.

Es können auch mehrere - gleiche oder verschiedene -geschützte Amino- und/oder Hydroxygruppen im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an einer anderen Stelle des Moleküls durchgeführt worden ist. Typisch für solche Gruppen sind insbesondere unsubstituierte oder substituierte Acyl-, Aryl- (z.B. DNP), Aralkoxymethyl- (z.B. BOM) oder Aralkyl-gruppen (z.B. Benzyl, 4-Nitrobenzyl, Triphenylmethyl). Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren im weitesten Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie POA; Alkoxycarbonyl wie Methoxycarbonyl, ETOC, 2,2,2-Trichlorethoxycarbonyl, IPOC, BOC, 2-Jodethoxycarbonyl; Aralkyloxycarbonyl wie CBZ, 4-Methoxybenzyloxycarbonyl, FMOC. Bevorzugte Aminoschutzgruppen sind BOC, DNP und BOM, ferner CBZ, FMOC, Benzyl und Acetyl.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an einer anderen Stelle des Moleküls durchgeführt worden ist. Typisch für solche Gruppen sind die oben genannten unsubstituierten oder substituierten Aryl-, Aralkyl- oder Acylgruppen, ferner auch Alkylgruppen. Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. tert.-Butyl, Benzyl, p-Nitrobenzoyl, p-Toluolsulfonyl und Acetyl, wobei Benzyl und Acetyl besonders bevorzugt sind.

Die als Ausgangsstoffe zu verwendenden funktionellen Derivate der Verbindungen der Formel I können nach üblichen Methoden der Aminosäure- und Peptidsynthese hergestellt werden, wie sie z.B. in den genannten Standardwerken und Patentanmeldungen beschrieben sind, z.B. auch nach der Festphasenmethode nach Merrifield.

Das In-Freiheit-Setzen der Verbindungen der Formel I aus ihren funktionellen Derivaten gelingt - je nach der benutzten Schutzgruppe - z.B. mit starken Säuren, zweckmäßig mit Trifluoressigsäure oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich.

Als inerte Lösungsmittel eignen sich vorzugsweise organische, beispielsweise Carbonsäuren wie Essigsäure, Ether wie Tetrahydrofuran oder Dioxan, Amide wie DMF, halogenierte Kohlenwasserstoffe wie Dichlormethan, ferner auch Alkohole wie Methanol, Ethanol oder Isopropanol sowie Wasser. Ferner kommen Gemische der vorgenannten Lösungsmittel in Frage. Trifluoressigsäure wird vorzugsweise im Überschuß ohne Zusatz eines weiteren Lösungsmittels verwendet, Perchlorsäure in Form eines Gemisches aus Essigsäure und 70%iger Perchlorsäure im Verhältnis 9:1. Die Reaktionstemperaturen für die Spaltung liegen zweckmäßig zwischen etwa 0 und etwa 50°; vorzugsweise arbeitet man zwischen 15 und 30° (Raumtemperatur).

Die BOC-Gruppe kann z.B. bevorzugt mit 40%iger Trifluoressigsäure in Dichlormethan oder mit etwa 3 bis 5 n HCl in Dioxan bei 15-30° abgespalten weden, die FMOC-Gruppe mit einer etwa 5-20%igen Lösung von Dimethylamin, Diethylamin oder Piperidin in DMF bei 15-30°. Eine Abspaltung der DNP-Gruppe gelingt z.B. auch mit einer etwa 3-10%igen Lösung von 2-Mercaptoethanol in DMF/Wasser bei 15-30°.

Hydrogenolytisch entfernbare Schutzgruppen (z.B. BOM, CBZ oder Benzyl) können z.B. durch Behandeln mit Wasserstoff in Gegenwart eines Katalysators (z.B. eines Edelmetallkatalysators wie Palladium, zweckmäßig auf einem Träger wie Kohle) abgespalten werden. Als Lösungsmittel eignen sich dabei die oben angegebenen, insbesondere z.B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt. Eine Hydrogenolyse der CBZ-Gruppe gelingt z.B. gut an 5-10%igem Pd-C in Methanol bei 20-30°.

Verbindungen der Formel I können auch durch direkte Kondensation (Peptidsynthese) aus einer Carbonsäure- (Formel II) und einer Hydroxy- bzw. Aminokomponente (Formel III) erhalten werden. Als Carbonsäurekomponenten eignen sich z.B. solche der Teilformeln (a) X-OH, (b) X-W-CR$^1$R$^2$-COOH (c) X-W-CR$^1$R$^2$-CO-Y-OH oder (d) H-W-CR$^1$R$^2$-CO-Y-NH-CHR$^4$-CR$^5$-CH$_2$-(CR$^6$R$^7$)$_r$-S(O)$_t$-CH$_2$-COOH, als Hydroxy- bzw. Aminokomponenten solche der Teilformeln (a) HW-CR$^1$R$^2$-CO-Y-NH-CHR$^4$-CR$^5$-CH$_2$-(CR$^6$R$^7$)$_r$-S(O)$_t$-CH$_2$CO-V-R$^3$ (worin W NH oder O bedeutet), (b) H-Y-NH-CHR$^4$-CR$^5$-CH$_2$-(CR$^6$R$^7$)$_r$-S(O)$_t$-CH$_2$CO-V-R$^3$ (c) H$_2$N-CHR$^4$-CR$^5$-CH$_2$-(CR$^6$R$^7$)$_r$-S(O)$_t$-CH$_2$CO-V-R$^3$ oder (d) H-V-R$^3$. Dabei arbeitet man zweckmäßig nach üblichen Methoden der Peptid-Synthese, wie sie z.B. in Houben-Weyl, 1.c., Band 15/II, Seiten 1-806 (1974) beschrieben sind; diese Methoden können, falls W = O ist, auch auf die Kondensation gemäß (a) übertragen werden, wobei eine Esterbindung entsteht.

Die Reaktion gelingt vorzugsweise in Gegenwart eines Dehydratisierungsmittels, z.B. eines Carbodiimids wie DCCI oder Dimethylaminopropylethyl-carbodiimid, ferner Propanphosphonsäureanhydrid (vgl. Angew.Chem. 92, 129 (1980)), Diphenylphosphorylazid oder 2-Ethoxy-N-ethoxycarbonyl-1,2-dihydrochinolin, in einem inerten Lösungsmittel, z.B. einem halogenierten Kohlenwasserstoff wie Dichlormethan, einem Ether wie THF oder Dioxan, einem Amid wie DMF oder Dimethylacetamid, einem Nitril wie Acetonitril, bei Temperaturen zwischen etwa -10 und 40, vorzugsweise zwischen 0 und 30°.

An Stelle von II bzw. III können auch geeignete reaktionsfähige Derivate dieser Stoffe in die Reaktion eingesetzt werden, z.B. solche, in denen reaktive Gruppen intermediär durch Schutzgruppen blockiert sind. Die Säurederivate II können z.B. in Form ihrer aktivierten Ester verwendet werden, die zweckmäßig in situ gebildet werden, z.B. durch Zusatz von HOBt oder N-Hydroxysuccinimid.

Die Ausgangsstoffe der Formeln II und III sind großenteils bekannt. Sofern sie nicht bekannt sind, können sie nach bekannten Methoden, z.B. den oben angegebenen Methoden der Kondensation und der Abspaltung von Schutzgruppen hergestellt werden.

Gewünschtenfalls kann in einer Verbindung der Formel I eine funktionell abgewandelte Amino- und/oder Hydroxygruppe durch Solvolyse oder Hydrogenolyse nach einer der oben beschriebenen Methoden in Freiheit gesetzt werden.

So kann z. B. eine Verbindung der Formel I, die eine R$^{11}$-C$_x$H$_{2x}$-O-CO-NH-, eine AcNH-, oder eine AOOC-Gruppe enthält, in die entsprechende Verbindung der Formel I umgewandelt werden, die stattdessen eine H$_2$N- oder eine HOOC-Gruppe enthält, zweckmäßig durch selektive Solvolyse nach einer der oben angegebenen

EP 0 464 517 A2

Methoden. AOOC-Gruppen können z.B. mit NaOH oder KOH in Wasser-Dioxan bei Temperaturen zwischen 0 und 40°, vorzugsweise 10 und 30°, verseift werden.

Es ist auch möglich, eine Verbindung der Formel I, die eine freie primäre oder sekundäre Aminogruppe enthält, zu acylieren. So kann man insbesondere Verbindungen der Formel I, in denen $R^8$ H bedeutet, mit acylierenden Mitteln der Formel X-Cl (worin X von H verschieden ist) umsetzen, zweckmäßig in Gegenwart eines inerten Lösungsmittels wie THF und/oder einer Base wie Pyridin oder Triethylamin bei Temperaturen zwischen -10 und +30°.

Weiterhin können Ketoverbindungen der Formel I ($R^5$ = O) zu Verbindungen der Formel I ($R^5$ = (H, OH)) reduziert werden, beispielsweise mit einem komplexen Metallhydrid wie $NaBH_4$, das nicht gleichzeitig die Peptid-Carbonylgruppen reduziert, in einem inerten Lösungsmittel wie Methanol bei Temperaturen zwischen etwa -10 und +30°.

Ketoverbindungen der Formel I ($R^5$ = O) können auch durch reduktive Aminierung in Verbindungen der Formel I ($R^5$ = H, $NH_2$) übergeführt werden. Man kann ein- oder mehrstufig reduktiv aminieren. So kann man z.B. die Ketoverbindung mit Ammoniumsalzen, z.B. Ammoniumacetat, und $NaCNBH_3$ behandeln, vorzugsweise in einem inerten Lösungsmittel, z.B. einem Alkohol wie Methanol, bei Temperaturen zwischen etwa 0 und 50°, insbesondere zwischen 15 und 30°. Weiterhin ist es möglich, die Ketoverbindung zunächst mit Hydroxylamin in üblicher Weise in das Oxim zu überführen und dieses, z.B. durch katalytische Hydrierung an Raney-Nickel, zum Amin zu reduzieren.

Gewünschtenfalls kann ein Sulfid der Formel I, worin t = 0 ist, zum entsprechenden Sulfoxid (I, t = 1) oder Sulfon (I, t = 2) oxydiert werden, z.B. mit Wasserstoffperoxid oder einer Persäure wie m-Chlorperoxybenzoesäure oder einem Salz einer Persäure wie Magnesium-monoperoxyphthalat-hexa-hydrat, in einem inerten Lösungsmittel wie Chloroform oder THF bei Temperaturen zwischen etwa -10 und +20°. Zur Herstellung des Sulfoxids verwendet man zweckmäßig etwa stöchiometrische Mengen des Oxydationsmittels. Mit der zur Bildung des Sulfons berechneten oder mit einer überschüssigen Menge des Oxydationsmittels erhält man dagegen überwiegend das Sulfon.

Eine Base der Formel I kann mit einer Säure in das zugehörige Säureadditionssalz übergeführt werden. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Trifluoressigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methanoder Ethansulfonsäure, Ethandisulfonsäure, 2-Hydroxyethansulfonsäure, Benzolsulfonsäure, p-Toluolsulfonsäure, Naphthalinmono- und -disulfonsäuren, Laurylschwefelsäure. Salze mit physiologisch nicht unbedenklichen Säuren, z.B. Pikrate, können zur Isolierung und/oder Aufreinigung der Verbindungen der Formel I verwendet werden.

Die neuen Verbindungen der Formel I und ihre physiologisch unbedenklichen Salze können zur Herstellung pharmazeutischer Präparate verwendet werden, indem man sie zusammen mit mindestens einem Träger- oder Hilfsstoff und, falls erwünscht, zusammen mit einem oder mehreren weiteren Wirkstoff(en) in eine geeignete Dosierungsform bringt. Die so erhaltenen Zubereitungen können als Arzneimittel in der Human- oder Veterinärmedizin eingesetzt werden. Als Trägersubstanzen kommen organische oder anorganische Stoffe in Frage, die sich für die enterale (z.B. orale oder rektale) oder parenterale Applikation oder für eine Applikation in Form eines Inhalations-Sprays eignen und mit den neuen Verbindungen nicht reagieren, beispielsweise Wasser, pflanzliche Öle, Benzylalkohole, Polyethylenglykole, Glycerintriacetat und andere Fettsäureglyceride, Gelatine, Soja-lecithin, Kohlehydrate wie Lactose oder Stärke, Magnesiumstearat, Talk, Cellulose. Zur oralen Anwendung dienen insbesondere Tabletten, Dragees, Kapseln, Sirupe, Säfte oder Tropfen; von Interesse sind speziell Lacktabletten und Kapseln mit magensaftresistenten Überzügen bzw. Kapselhüllen. Zur rektalen Anwendung dienen Suppositorien, zur parenteralen Applikation Lösungen, vorzugsweise ölige oder wässerige Lösungen, ferner Suspensionen, Emulsionen oder Implantate. Für die Applikation als Inhalations-Spray können Sprays verwendet werden, die den Wirkstoff entweder gelöst oder suspendiert in einem Treibgasgemisch (z.B. Fluorchlorkohlenwasserstoffen) enthalten. Zweckmäßig verwendet man den Wirkstoff dabei in mikronisierter Form, wobei ein oder mehrere zusätzliche physiologisch verträgliche Lösungsmittel zugegen sein können, z.B. Ethanol. Inhalations-Lösungen können mit Hilfe üblicher Inhalatoren verabfolgt werden. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Konservierungs-, Stabilisierungs- und/oder Netzmittel, Emulgatoren, Salze zur Beeinflussung des osmotischen Druckes, Puffersubstanzen, Farb- und/oder Aromastoffe enthalten. Sie können, falls erwünscht, auch einen oder mehrere weitere Wirkstoffe enthalten, z.B. ein oder mehrere Vitamine.

Die erfindungsgemäßen Substanzen werden in der Regel in Analogie zu anderen bekannten, im Handel befindlichen Peptiden, insbesondere aber in Analogie zu den in der EP-A-249096 beschriebenen Verbindungen verabreicht, vorzugsweise in Dosierungen zwischen etwa 10 mg und 1 g, insbesondere zwischen 50 und 500 mg pro Dosierungseinheit. Die tägliche Dosierung liegt vorzugsweise zwischen etwa 0,2 und 20 mg/kg, insbesondere 1 und 10 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die parenterale Applikation ist bevorzugt. Renin-abhängige Hypertension und Hyperaldosteronismus können wirksam behandelt werden durch Verabfolgung von Dosierungen zwischen insbesondere etwa 0,2 und 20, vorzugsweise zwischen 1 und 10 mg/kg Körpergewicht. Für diagnostische Zwecke können die neuen Verbindungen zweckmäßig in Einzeldosen zwischen etwa 0,1 und 10 mg/kg Körpergewicht verabreicht werden.

Vor- und nachstehend sind alle Temperaturen in °C angegeben. In den nachfolgenden Beispielen bedeutet "übliche Aufarbeitung": Man gibt, falls erforderlich, Wasser hinzu, stellt je nach Konstitution des Endprodukts auf pH-Werte zwischen 2 und 8 ein, extrahiert mit Ethylacetat oder Dichlormethan, trennt ab, trocknet die organische Phase über Natriumsulfat, dampft ein und reinigt durch Chromatographie an Kieselgel und/oder Kristallisation. TFA = Trifluoracetat.

Beispiel 1

Man löst 1 g 6S-[2RS-Benzyl-3-tert.-butylsulfonyl-propionyl-(L-(N-imi)-benzyloxymethyl-histidyl)-amino]-7-cyclohexyl-5S-hydroxy-3-thia-heptansäure-(N-isobutyl-amid) [= 6S-[2RS-Benzyl-3-tert.-butylsulfonyl-propionyl-(imi-BOM-His)-amino]-7-cyclohexyl-5S-hydroxy-3-thia-heptansäure-(N-isobutyl-amid); erhältlich durch Reaktion von 3-BOC-4-cyclohexylmethyl-5-jodmethyl-2,2-dimethyl-oxazolidin (F. 89°) mit Thioglykolsäure in Gegenwart von NaH in DMF zu 3-BOC-5-(3-carboxy-2-thiapropyl)-4-cyclohexylmethyl-2,2-dimethyl-oxazolidin (F. 115-116°), Kondensation mit Isobutylamin zu 3-BOC-4-cyclohexylmethyl-5-(3-N-isobutylcarbamoyl-2-thiapropyl)-4-cyclohexylmethyl-2,2-dimethyl-oxazolidin, Hydrolyse zu 6S-Amino-7-cyclohexyl-5S-hydroxy-3-thia-heptansäure-(N-isobutyl-amid) und Kondensation mit 2-RS-Benzyl-3-tert.-Butylsulfonyl-propionyl-(imi-BOM-His)-OH analog Beispiel 4] in 26 ml Ethanol, hydriert an 0,4 g 10%ig. Pd-C bei 20° und 1 bar bis zum Stillstand der $H_2$-Aufnahme, filtriert, dampft ein und erhält nach chromatographischer Reinigung 6S-(2RS-Benzyl-3-tert.-butylsulfonyl-propionyl-His-amino)-7-cyclohexyl-5S-hydroxy-3-thia-heptansäure-(N-isobutyl-amid), Hydrochlorid, 2 Isomere, F. 136-137° und F. 99-100°.

Beispiel 2

Analog Beispiel 1 erhält man aus 6S-[4-BOC-amino-piperi-dinocarbonyl-Pla-(imi-BOM-His)-amino]-7-cyclohexyl-5S-hydroxy-3-thia-heptansäure-(N-isobutyl-amid) durch Hydrogenolyse 6S-(4-BOC-amino-piperidinocarbonyl-Pla-His-amino)-7-cyclohexyl-5S-hydroxy-3-thia-heptansaüre-(N-isobutyl-amid), F. 149°.

Beispiel 3

Analog Beispiel 1 erhält man aus 6S-[4-BOC-amino-piperi-dinocarbonyl-Phe-(imi-BOM-His)-amino]-7-cyclohexyl-5S-hydroxy-3-thiaheptansäure-(N-isobutyl-amid) durch Hydrogenolyse 6S-(4-BOC-amino-piperidinocarbonyl-Phe-His-amino)-7-cyclohexyl-5S-hydroxy-3-thia-heptansäure-(N-isobutyl-amid), F. 116-117°.

Analog erhält man

6S-(4-BOC-amino-piperidinocarbonyl-Phe-His-amino)-7-cyclohexyl-5S-hydroxy-3-thia-heptansäure-(N-isobutyl-amid)-3,3-dioxid
6S-Acetyl-Cal-His-amino-7-cyclohexyl-5S-hydroxy-3-thia-heptansäure-methylester
6S-BOC-Bia-His-amino-7-cyclohexyl-5S-hydroxy-3-thia-heptansäure-methylester
7-Cyclohexyl-5S-hydroxy-6S-isopropylsulfonyl-Phe-His-amino-3-thia-heptansäure-methylester
7-Cyclohexyl-5S-hydroxy-6S-morpholinocarbonyl-Ada-His-amino-3-thia-heptansäure-methylester
7-Cyclohexyl-5S-hydroxy-6S-morpholinocarbonyl-Hph-His-amino-3-thia-heptansäure-methylester
7-Cyclohexyl-5S-hydroxy-6S-morpholinocarbonyl-Leu-His-amino-3-thia-heptansäure-methylester
7-Cyclohexyl-5S-hydroxy-6S-morpholinocarbonyl-Mal-His-amino-3-thia-heptansäure-methylester

7-Cyclohexyl-5S-hydroxy-6S-morpholinocarbonyl-Phe-His-amino-3-thia-heptansäure-methylester
7-Cyclohexyl-5S-hydroxy-6S-morpholinocarbonyl-Tia-His-amino-3-thia-heptansäure-methylester
7-Cyclohexyl-5S-hydroxy-6S-morpholinocarbonyl-Trp-His-amino-3-thia-heptansäure-methylester
7-Cyclohexyl-5S-hydroxy-6S-morpholinosulfonyl-Phe-His-amino-3-thia-heptansäure-methylester
6S-(4-BOC-amino-piperidinocarbonyl-Phe-His-amino)-7-cyclohexyl-5S-hydroxy-3-thia-heptansäure-methylester
6S-(4-BOC-amino-piperidinocarbonyl-Pla-His-amino)-7-cyclohexyl-5S-hydroxy-3-thia-heptansäure-methylester
6S-(4-BOC-amino-piperidinocarbonyl-Phe-His-amino)-7-cyclohexyl-5S-hydroxy-3-thia-heptansäure-methylester-3,3-dioxid
6S-(4-BOC-amino-piperidinocarbonyl-Pla-His-amino)-7-cyclohexyl-5S-hydroxy-3-thia-heptansäure-methylester-3,3-dioxid
7S-(4-BOC-amino-piperidinocarbonyl-Phe-His-amino)-8-cyclohexyl-6S-hydroxy-3-thia-octansäure-methylester
7S-(4-BOC-amino-piperidinocarbonyl-Pla-His-amino)-8-cyclohexyl-6S-hydroxy-3-thia-octansäure-methylester
7S-(4-BOC-amino-piperidinocarbonyl-Phe-His-amino)-8-cyclohexyl-6S-hydroxy-3-thia-octansäure-methylester-3,3-dioxid
7S-(4-BOC-amino-piperidinocarbonyl-Pla-His-amino)-8-cyclohexyl-6S-hydroxy-3-thia-octansäure-methylester-3,3-dioxid
7-Cyclohexyl-5S-hydroxy-6S-morpholinocarbonyl-Phe-His-amino3-thia-heptansäure-methylester-3,3-dioxid
7-Cyclohexyl-5S-hydroxy-6S-morpholinocarbonyl-Pla-His-amino-3-thia-heptansäure-methylester-3,3-dioxid
6S-(2-RS-Benzyl-3-tert.-butylsulfonyl-propionyl-His-amino)-7-cyclohexyl-5S-hydroxy-3-thia-heptansäure-methylester-3,3-dioxid.

## Beispiel 4

Ein Gemisch von 992 mg 6S-[4-BOC-amino-piperidinocarbonylamino-L-phenylalanyl-L-(N-(imi)-2,4-dinitrophenylhistidyl)-amino]-7-cyclohexyl-5S-hydroxy-3-thia-heptansäure-(N-isobutylamid) [= 6S-[4-BOC-amino-piperidinocarbonyl-Phe-(imi-DNP-His)-amino]-7-cyclohexyl-5S-hydroxy-3-thia-heptansäure-(N-isobutyl-amid); erhältlich über 6S-BOC-(imi-DNP-His)-amino-7-cyclohexyl-5S-hydroxy-3-thiaheptansäure-(N-isobutyl-amid) (F. 95-96°)], 2 g 2-Mercaptoethanol, 20 ml DMF und 20 ml Wasser wird unter Rühren bei 20° mit wässeriger $Na_2CO_3$-Lösung auf pH 8 eingestellt und noch 2 Std. bei 20° gerührt. Nach üblicher Aufarbeitung erhält man 6S-(4-BOC-amino-piperidinocarbonyl-Phe-His-amino)-7-cyclohexyl-5S-hydroxy-3-thia-heptansäure-(N-isobutyl-amid), F. 116-117°.

## Beispiel 5

Analog Beispiel 1 erhält man durch Hydrogenolyse von 6S-(4-BOC-amino-piperidinocarbonyl-Phe-(N5-CBZ-Lys)-amino)-7-cyclohexyl-5S-hydroxy-3-thia-heptansäure-(N-2-phenylethylamid) das 6S-(4-BOC-amino-piperidinocarbonyl-Phe-Lys-amino)-7-cyclohexyl-5S-hydroxy-3-thia-heptansäure-(N-2-phenylethylamid).

## Beispiel 6

Eine Lösung von 3,96 g 7-Cyclohexyl-5S-hydroxy-6S-H-(S-Me-Cys)-amino-3-thia-heptansäure-(N-isobutyl-amid) [erhältlich durch Kondensation von 6S-Amino-7-cyclohexyl-5S-hydroxy-3-thia-heptansäure-(N-isobutyl-amid) mit BOC-(S-Me-Cys)-OH zu 6S-[BOC-(S-Me-Cys)-amino]-7-cyclohexyl-5S-hydroxy-3-thia-heptansäure-(N-isobutylamid) (F. 107°) und Abspaltung der BOC-Gruppe] in 60 ml Dichlormethan wird mit 1,01 g N-Methylmorpholin versetzt. Unter Rühren gibt man 3,79 g 4-BOC-amino-piperidinocarbonyl-Phe-OH, 1,35 g HOBt und eine Lösung von 2,06 g DCCI in 50 ml Dichlormethan hinzu, rührt 12 Std. bei 0-5 °, filtriert den ausgeschiedenen Dicyclohexylharnstoff ab und dampft das Filtrat ein. Nach üblicher Aufarbeitung erhält man 6S-[4-BOC-amino-piperidinocarbonyl-Phe-(S-Me-Cys)-amino]-7-cyclohexyl-5S-hydroxy-3-thia-heptansäure-(N-isobutyl-amid), F. 101-102°.

## Beispiel 7

Analog Beispiel 6 erhält man mit 4-BOC-amino-piperidinocarbonyl-Pla-OH (F. 123-124°) das 6S-[4-BOC-amino-piperidinocarbonyl-Pla-(S-Me-Cys)-amino]-7-cylcohexyl-5S-hydroxy-3-thia-heptansäure-(N-isobutyl-amid), F. 172-173°.

Beispiel 8

Analog Beispiel 6 erhält man aus 4-BOC-amino-piperidinocarbonyl-Phe-βAla-OH und 6S-Amino-7-cyclo-hexyl-5S-hydroxy-3-thia-heptansäure-(N-isobutyl-amid) das 6S-(4-BOC-amino-piperidinocarbonyl-Phe-βAla-amino)-7-cyclohexyl-5S-hydroxy-3-thia-heptansäure-(N-isobutyl-amid), F. 100-101°.

Analog erhält man mit 6S-Amino-7-cyclohexyl-5S-hydroxy-3-thiaheptansäuremethylester bzw. mit dessen 3,3-Dioxid:

6S-(4-BOC-amino-piperidinocarbonyl-Phe-βAla-amino)-7-cyclohexyl-5S-hydroxy-3-thia-heptansäure-methylester, F.140-141° 6S-(4-BOC-amino-piperidinocarbonyl-Phe-βAla-amino)-7-cyclohexyl-5S-hydroxy-3-thia-heptansäure-methylester-3,3-dioxid, F. 113-114°.

Beispiel 9

Analog Beispiel 6 erhält man mit 8-Cyclohexyl-6S-hydroxy-7S-H-(S-Me-Cys)-amino-3-thia-octansäure-(N-isobutyl-amid) [erhältlich aus 3-BOC-4-cyclohexylmethyl-5-(2-jodethyl)-2,2-dimethy-oxazolidin über 3-BOC-4-cyclohexylmethyl-5-(4-carboxy-3-thia-butyl)-2,2-dimethyl-oxazolidin, 3-BOC-4-cyclohexylmethyl-5-(4-isobutylaminocarbonyl-3-thia-butyl)-2,2-dimethyl-oxazolidin (F. 61-62°) und 8-Cyclohexyl-6S-hydroxy-7S-BOC-(S-Me-Cys)-amino-3-thia-octansäure-(N-isobutyl- amid) (F. 124-125°)] das 7S-[4-BOC-amino-piperidino-carbonyl-Phe-(S-Me-Cys)-amino]-8-cyclohexyl-6S-hydroxy-3-thia-octansäure-(N-isobutyl-amid), F. 149-150°.

Beispiel 10

Analog Beispiel 6 erhält man aus 4-BOC-amino-piperidinocarbonyl-Pla-OH und 7-Cyclohexyl-5S-hydroxy-6S-H-(S-Me-Cys)-amino-3-thia-heptansäure-methylester [erhältlich über 3-BOC-4-cyclohexylmethyl-5-jodmethyl-2,2-dimethyl-oxazolidin (F. 67°) und 3-BOC-4-cyclohexylmethyl-5-(3-methoxycarbonyl-2-thia-propyl)-2,2-dimethyl-oxazolidin (F. 67°)] den 6S-[4-BOC-amino-piperidino-carbonyl-Pla-(S-Me-Cys)-amino]-7-cyclo-hexyl-5S-hydroxy-3-thia-heptansäure-methylester, F. 104-105°.

Beispiel 11

Analog Beispiel 10 erhält man mit 4-BOC-amino-piperidinocarbonyl-Phe-OH den 6S-[4-BOC-amino-piperidinocarbonyl-Phe-(S-Me-Cys)-amino]-7-cyclohexyl-5S-hydroxy-3-thia-heptansäure-methylester, F. 90-91°.

Beispiel 12

Analog Beispiel 10 erhält man mit Morpholinocarbonyl-Phe-OH bzw. mit Morpholinocarbonyl-Pla-OH:
7-Cyclohexyl-5S-hydroxy-6S-[morpholinocarbonyl-Phe-(S-Me-Cys)-amino]-3-thia-heptansäure-methylester.
7-Cyclohexyl-5S-hydroxy-6S-[morpholinocarbonyl-Pla-(S-Me-Cys)-amino]-3-thia-heptansäure-methylester.

Beispiel 13

Analog Beispiel 6 erhält man aus Morpholinocarbonyl-Pla-OH und 8-Cyclohexyl-6S-hydroxy-7S-H-(S-Me-Cys)-amino-3-thia-octansäure-(N-isobutyl-amid) das 8-Cyclohexyl-6S-hydroxy-7S-[morpholinocarbonyl-Pla-(S-Me-Cys)-amino]-3-thia-octansäure-(N-isobutyl-amid), F. 97-98°.

Beispiel 14

Analog Beispiel 13 erhält man mit 4-BOC-amino-piperidinocarbonyl-Pla-OH das 7S-[4-BOC-amino-piperidinocarbonyl-Pla-(S-Me-Cys)-amino]-8-cyclohexyl-6S-hydroxy-3-thia-octansäure-(N-isobutyl-amid), F. 171-172°.

Beispiel 15

Analog Beispiel 6 erhält man aus 4-BOC-amino-piperidinocarbonyl-Pla-OH und 8-Cyclohexyl-6S-hydroxy-7S-H-(S-Me-Cys)-amino-3-thia-octansäure-methylester den 7S-[4-BOC-amino-piperidinocarbonyl-Pla-(S-Me-Cys)-amino]-8-cyclohexyl-6S-hydroxy-3-thia-octansäure-methylester, F. 58-59°.

Analog erhält man mit 4-BOC-amino-piperidinocarbonyl-Phe-OH den 7S-[4-BOC-amino-piperidinocarbonyl-Phe-(S-Me-Cys)-amino]-8-cyclohexyl-6S-hydroxy-3-thia-octansäure-methylester, F 175-176°.

Beispiel 16

Analog Beispiel 6 erhält man aus Morpholinocarbonyl-Phe-(S-Me-Cys)-OH und 8-Cyclohexyl-6S-hydroxy-7S-amino-3-thia-octansäure-methylester den 8-Cyclohexyl-6S-hydroxy-7S-morpholinocarbonyl-Phe-(S-Me-Cys)-amino-3-thia-octansäure-methylester, F. 135-136°.

Beispiel 17

Analog Beispiel 6 erhält man aus 3-Phenylpropionsäure und 7-Cyclohexyl-5S-hydroxy-6S-(S-Me-Cys)-amino-3-thia-heptansäure-methylester-3,3-dioxid das 7-Cyclohexyl-5S-hydroxy-6S-[3-phenylpropionyl-(S-Me-Cys)-amino]-3-thia-heptansäure-methylester-3,3-dioxid, F. 132-133°.

Analog erhält man:

mit Phenoxyessigsäure (POA-H) das 7-Cyclohexyl-5S-hydroxy-6S-[POA-(S-Me-Cys)-amino]-3-thia-heptansäure-methylester-3,3-dioxid, F. 138-139°;
mit BOC-Phe-OH das 6S-BOC-Phe-(S-Me-Cys)-amino-7-cyclohexyl-5S-hydroxy-3-thia-heptansäure-methylester-3,3-dioxid, F. 154-155°;
mit 4-BOC-amino-piperidinocarbonyl-Phe-OH das 6S-[4-BOC-amino-piperidinocarbonyl-Phe-(S-Me-Cys)-amino]-7-cyclohexyl-5S-hydroxy-3-thia-heptansäure-methylester-3,3-dioxid, F 139-140°.
mit 4-BOC-amino-piperidinocarbonyl-Pla-OH das 6S-[4-BOC-amino-piperidinocarbonyl-Pla-(S-Me-Cys)-amino]-7-cyclohexyl-5S-hydroxy-3-thia-heptansäure-methylester-3,3-dioxid, F. 178-179°.
mit Morpholinocarbonyl-Phe-OH das 7-Cyclohexyl-5S-hydroxy-6S-morpholinocarbonyl-Phe-(S-Me-Cys)-amino-3-thia-heptansäure-methylester-3,3-dioxid, F. 139-141°.

Beispiel 18

Analog Beispiel 6 erhält man aus 4-BOC-amino-piperidinocarbonyl-Pla-OH und 8-Cyclohexyl-6S-hydroxy-7S-(S-Me-Cys)-amino-3-thia-octansäure-methylester-3,3-dioxid das 7S-[4-BOC-amino-piperidinocarbonyl-Pla-(S-Me-Cys)-amino]-8-cyclohexyl-6S-hydroxy-3-thia-octansäure-methylester-3,3-dioxid, F. 167-168°.

Analog erhält man:

mit 4-BOC-amino-piperidinocarbonyl-Phe-OH das 7S-[4-BOC-amino-piperidinocarbonyl-Phe-(S-Me-Cys)-amino]-8-cyclohexyl-6S-hydroxy-3-thia-octansäure-methylester-3,3-dioxid, F. 151-152°;
mit Morpholinocarbonyl-Phe-OH das 8-Cyclohexyl-6S-hydroxy-7S-morpholinocarbonyl-Phe-(S-Me-Cys)-amino-3-thia-octansäure-methylester-3,3-dioxid, F. 155-156°;
mit POA das 8-Cyclohexyl-6S-hydroxy-7S-POA-(S-Me-Cys)-amino-3-thia-octansäure-methylester-3,3-dioxid, F. 134-135°;
mit Indol-2-carbonsäure das 8-Cyclohexyl-6S-hydroxy-7S-[2-Indolylcarbonyl-(S-Me-Cys)-amino]-3-thia-octansäure-methylester-3,3-dioxid, F. 175-176°;
mit Morpholinocarbonyl-Pla-OH das 7S-[4-BOC-amino-piperidinocarbonyl-Pla-(S-Me-Cys)-amino]-3-thia-octansäure-methylester-3,3-dioxid, F. 112-113°.

Beispiel 19

Analog Beispiel 6 erhält man aus 4-BOC-amino-piperidinocarbonyl-Phe-OH und 7-Cyclohexyl-5R,S-hydroxy-6-H-(S-Me-Cys)-amino-3-thia-heptansäure-(N-isobutyl-amid)-3,3-dioxid das 6-[4-BOC-amino-piperidinocarbonyl-Phe-(S-Me-Cys-amino]-7-cyclohexyl-5R,S-hydroxy-3-thia-heptansäure-(N-isobutylamid)-3,3-dioxid, F. 118-119°.
Analog erhält man aus 7-Cyclohexyl-5S-hydroxy-6-H-(S-Me-Cys)amino-3-thia-heptansäure-(N-isobutylamid)-3,3-dioxid das entsprechende 5S-Diastereomere, F. 154-155°.
Analog erhält man mit 4-BOC-amino-piperidinocarbonyl-Pla-OH das 6-[4-BOC-amino-piperidinocarbonyl-Pla-(S-Me-Cys)-amino]-7-cyclohexyl-5S-hydroxy-3-thia-heptansäure-(N-isobutylamid) -3, 3-dioxid, F. 96-97°.

Beispiel 20

Analog Beispiel 6 erhält man aus 4-BOC-amino-piperidinocarbonyl-Phe-Leu-OH und 6S-Amino-7-cyclo-

hexyl-5S-hydroxy-3-thia-heptansäure-(N-isobutyl-amid)-3,3-dioxid das 6S-(4-BOC-amino-piperidinocarbonyl-Phe-Leu-amino)-7-cyclohexyl-5S-hydroxy-3-thia-heptansäure-(N-isobutyl-amid)-3,3-dioxid.

Analog erhält man mit den entsprechenden Verbindungen des Typs 4-BOC-amino-piperidinocarbonyl-Phe-Y-OH (Y = Ala, βAla, Met, Nle, Nva, 3-Pya, Tia bzw. 2-Tiz):

6S-(4-BOC-amino-piperidinocarbonyl-Phe-Ala-amino)-7-cyclohexyl-5S-hydroxy-3-thia-heptansäure-(N-isobutyl-amid)-3,3-dioxid

6S-(4-BOC-amino-piperidinocarbonyl-Phe-βAla-amino)-7-cyclohexyl-5S-hydroxy-3-thia-heptansäure-(N-isobutyl-amid)-3,3-dioxid

6S-(4-BOC-amino-piperidinocarbonyl-Phe-Met-amino)-7-cyclohexyl-5S-hydroxy-3-thia-heptansäure-(N-isobutyl-amid)-3,3-dioxid

6S-(4-BOC-amino-piperidinocarbonyl-Phe-Nle-amino)-7-cyclohexyl-5S-hydroxy-3-thia-heptansäure-(N-isobutyl-amid)-3,3-dioxid

6S-(4-BOC-amino-piperidinocarbonyl-Phe-Nva-amino)-7-cyclohexyl-5S-hydroxy-3-thia-heptansäure-(N-isobutyl-amid)-3,3-dioxid

6S-(4-BOC-amino-piperidinocarbonyl-Phe-(3-Pya)-amino)-7-cyclohexyl-5S-hydroxy-3-thia-heptansäure-(N-isobutylamid)-3,3-dioxid

6S-(4-BOC-amino-piperidinocarbonyl-Phe-Tia-amino)-7-cyclohexyl-5S-hydroxy-3-thia-heptansäure-(N-isobutyl-amid)-3,3-dioxid

6S-(4-BOC-amino-piperidinocarbonyl-Phe-(2-Tiz)-amino)-7-cyclo-  hexyl-5S-hydroxy-3-thia-heptansäure-(N-isobutylamid)-3,3-dioxid.


Beispiel 21


Analog Beispiel 6 erhält man aus Morpholinocarbonyl-Phe-(S-Me-Cys)-OH und 6S-Amino-7-cyclohexyl-5S-hydroxy-3-thia-heptansäure-(N-methyl-amid) das 7-Cyclohexyl-5S-hydroxy-6S-morpholinocarbonyl-Phe-(S-Me-Cys)-amino-3-thia-heptansäure-(N-methyl-amid).

Analog erhält man mit 6S-Amino-7-cyclohexyl-5S-hydroxy-3-thia-heptansäure-(N-propyl-amid), -isopropylester, -(N-Methyl-amid)-3,3-dioxid bzw. -(N-propyl-amid)-3,3-dioxid:

7-Cyclohexyl-5S-hydroxy-6S-morpholinocarbonyl-Phe-(S-Me-Cys)-amino-3-thia-heptansäure-(N-propyl-amid)

7-Cyclohexyl-5S-hydroxy-6S-morpholinocarbonyl-Phe-(S-Me-Cys)-amino-3-thia-heptansäure-isopropylester

7-Cyclohexyl-5S-hydroxy-6S-morpholinocarbonyl-Phe-(S-Me-Cys)-amino-3-thia-heptansäure-(N-methyl-amid)-3,3-dioxid

7-Cyclohexyl-5S-hydroxy-6S-morpholinocarbonyl-Phe-(S-Me-Cys)-amino-3-thia-heptansäure-(N-propyl-amid)-3,3-dioxid.

Analog erhält man mit Morpholinocarbonyl-Pla-(S-Me-Cys)-OH:

7-Cyclohexyl-5S-hydroxy-6S-morpholinocarbonyl-Pla-(S-Me-Cys)-amino-3-thia-heptansäure-(N-methyl-amid)

7-Cyclohexyl-5S-hydroxy-6S-morpholinocarbonyl-Pla-(S-Me-Cys)-amino-3-thia-heptansäure-(N-propyl-amid)

7-Cyclohexyl-5S-hydroxy-6S-morpholinocarbonyl-Pla-(S-Me-Cys)-amino-3-thia-heptansäure-isopropylester

7-Cyclohexyl-5S-hydroxy-6S-morpholinocarbonyl-Pla-(S-Me-Cys)-amino-3-thia-heptansäure-(N-methyl-amid)-3,3-dioxid

7-Cyclohexyl-5S-hydroxy-6S-morpholinocarbonyl-Pla-(S-Me-Cys)-amino-3-thia-heptansäure-(N-propyl-amid)-3,3-dioxid.


Beispiel 22


Analog Beispiel 6 erhält man aus 4-BOC-amino-piperidinocarbonyl-Phe-Leu-OH bzw. 4-BOC-amino-piperidinocarbonyl-Pla-Leu-OH mit 6S-Amino-7-cyclohexyl-5S-hydroxy-3-thia-heptansäure-methylester-3,3-dioxid bzw. mit 7S-Amino-8-cylohexyl-6S-hydroxy-3-thia-octansäure-methylester-3,3-dioxid:

6S-(4-BOC-amino-piperidinocarbonyl-Phe-Leu-amino)-7-cyclohexyl-5S-hydroxy-3-thia-heptansäure-methylester-3,3-dioxid

6S-(4-BOC-amino-piperidinocarbonyl-Pla-Leu-amino)-7-cyclohexyl-5S-hydroxy-3-thia-heptansäure-methylester-3,3-dioxid

7S-(4-BOC-amino-piperidinocarbonyl-Phe-Leu-amino)-8-cyclohexyl-6S-hydroxy-3-thia-octansäure-methylester-3,3-dioxid

7S-(4-BOC-amino-piperidinocarbonyl-Pla-Leu-amino)-8-cyclohexyl-6S-hydroxy-3-thia-octansäure-methylester-3,3-dioxid.

Beispiel 23

Analog Beispiel 6 erhält man aus 2-Benzyl-3-tert.-butylsulfonyl-propionyl-(S-Me-Cys)-OH mit 6S-Amino-7-cyclohexyl-5S-hydroxy-3-thia-heptansäure-methylester-3,3-dioxid, 7S-Amino-8-cyclohexyl-6S-hydroxy-3-thia-octansäure-methylester bzw. deren 3-Oxid oder 3,3-Dioxid:
6S-[2-Benzyl-3-tert.-butylsulfonyl-propionyl-(S-Me-Cys)-amino]-7-cyclohexyl-5S-hydroxy-3-thia-heptansäure-methylester-3,3-dioxid
7S-[2-Benzyl-3-tert.-butylsulfonyl-propionyl-(S-Me-Cys)-amino]-8-cyclohexyl-6S-hydroxy-3-thia-octansäure-methylester
7S-[2-Benzyl-3-tert.-butylsulfonyl-propionyl-(S-Me-Cys)-amino]-8-cyclohexyl-6S-hydroxy-3-thia-octansäure-methylester-3-oxid
7S-[2-Benzyl-3-tert.-butylsulfonyl-propionyl-(S-Me-Cys)-amino]-8-cyclohexyl-6S-hydroxy-3-thia-octansäure-methylester-3,3-dioxid.

Beispiel 24

Analog Beispiel 6 erhält man aus

4-BOC-amino-piperidinocarbonyl-Phe-βAla-OH
4-BOC-amino-piperidinocarbonyl-Pla-βAla-OH
Morpholinocarbonyl-Phe-βAla-OH
Morpholinocarbonyl-Pla-βAla-OH
Morpholinocarbonyl-Phe-(S-Me-Cys)-OH
Morpholinocarbonyl-Pla-(S-Me-Cys)-OH
mit 7S-Amino-8-cyclohexyl-6S-hydroxy-3-thia-octansäure-methylester bzw. dessen 3,3-dioxid:
7S-(4-BOC-amino-piperidinocarbonyl-Phe-βAla-amino)-8-cyclohexyl-6S-hydroxy-3-thia-octansäure-methylester
7S-(4-BOC-amino-piperidinocarbonyl-Pla-βAla-amino)-8-cyclohexyl-6S-hydroxy-3-thia-octansäure-methylester
8-Cyclohexyl-6S-hydroxy-7S-(morpholinocarbonyl-Phe-βAla-amino)-6S-hydroxy-3-thia-octansäure-methylester
8-Cyclohexyl-6S-hydroxy-7S-(morpholinocarbonyl-Pla-βAla-amino)-6S-hydroxy-3-thia-octansäure-methylester
8-Cyclohexyl-6S-hydroxy-7S-[morpholinocarbonyl-Phe-(S-Me-Cys)-amino]-6S-hydroxy-3-thiaoctansäure-methyl- ester
8-Cyclohexyl-6S-hydroxy-7S-[morpholinocarbonyl-Pla-(S-Me-Cys)-amino]-6S-hydroxy-3-thiaoctansäure-methyl- ester
7S-(4-BOC-amino-piperidinocarbonyl-Phe-βAla-amino)-8-cyclohexyl-6S-hydroxy-3-thia-octansäure-methylester-3,3-dioxid
7S-(4-BOC-amino-piperidinocarbonyl-Pla-βAla-amino)-8-cyclohexyl-6S-hydroxy-3-thia-octansäure-methylester-3,3-dioxid
8-Cyclohexyl-6S-hydroxy-7S-(morpholinocarbonyl-Phe-βAlaamino)-6S-hydroxy-3-thia-octansäure-methylester-3,3-dioxid
8-Cyclohexyl-6S-hydroxy-7S-(morpholinocarbonyl-Pla-βAla-amino)-6S-hydroxy-3-thiaoctansäure-methylester- 3,3-dioxid
8-Cyclohexyl-6S-hydroxy-7S-[morpholinocarbonyl-Phe-(S-Me-Cys)-amino]-6S-hydroxy-3-thia-octansäure-methyl-ester-3,3-dioxid
8-Cyclohexyl-6S-hydroxy-7S-[morpholinocarbonyl-Pla-(S-Me-Cys)-amino]-6S-hydroxy-3-thia-octansäure-methylester-3,3-dioxid, F. 112° (Zers.).

Beispiel 25

Analog Beispiel 6 erhält man aus 6S-(4-BOC-amino-piperidinocarbonyl-Phe-βAla-amino)-7-cyclohexyl-5S-hydroxy-3-thia-heptansäure und Isobutylamin das 6S-(4-BOC-amino-piperidinocarbonyl-Phe-βAla-amino)-7-cyclohexyl-5S-hydroxy-3-thia-heptansäure-(N-isobutylamid), F. 100-101°.

Beispiel 26

Eine Lösung von 1 g 6S-[4-BOC-amino-piperidinocarbonyl-Phe-(S-Me-Cys)-amino]-7-cyclohexyl-5S-hydroxy-3-thia-heptansäure-(N-isobutyl-amid) in 20 ml Dichlormethan und 20 ml Trifluoressigsäure wird 1 Std. bei 20° gerührt und dann eingedampft. Man erhält 6S-[4-Amino-piperidinocarbonyl-Phe-(S-Me-Cys)-amino]-7-cyclohexyl-5S-hydroxy-3-thia-heptansäure-(N-isobutyl-amid). TFA, F. 159-160°.

Analog erhält man aus den entsprechenden BOC-amino-derivaten mit Trifluoressigsäure:

6S-[4-Amino-piperidinocarbonyl-Phe-(S-Me-Cys)-amino]-7-cyclohexyl-5R-hydroxy-3-thia-heptansäure-(N-isobutyl-amid), TFA, F. 145° (Zers.)

6S-[4-Amino-piperidinocarbonyl-Pla-(S-Me-Cys)-amino]-7-cyclohexyl-5S-hydroxy-3-thia-heptansäure-(N-isobutyl-amid), TFA, F. 112° (Zers.)

6S-(4-Amino-piperidinocarbonyl-Phe-βAla-amino)-7-cyclohexyl-5S-hydroxy-3-thia-heptansäure-(N-isobutyl-amid), TFA, F. 141°

6S-(4-Amino-piperidinocarbonyl-Pla-βAla-amino)-7-cyclohexyl-5S-hydroxy-3-thia-heptansäure-(N-isobutyl-amid)

6S-(4-Amino-piperidinocarbonyl-Phe-His-amino)-7-cyclohexyl-5S-hydroxy-3-thia-heptansäure-(N-isobutyl-amid), Bis-TFA, F. 149-150°

6S-(4-Amino-piperidinocarbonyl-Pla-His-amino)-7-cyclohexyl-5S-hydroxy-3-thia-heptansäure-(N-isobutyl-amid), Bis-TFA, F. 125-126°

6S-[4-Amino-piperidinocarbonyl-Phe-(S-Me-Cys)-amino]-7-cyclohexyl-5S-hydroxy-3-thia-heptansäure-methylester, Hydrochlorid, F. 184-185°

6S-[4-Amino-piperidinocarbonyl-Pla-(S-Me-Cys)-amino]-7-cyclohexyl-5S-hydroxy-3-thia-heptansäure-methylester, Hydrochlorid, F. 124-125°

6S-(4-Amino-piperidinocarbonyl-Phe-His-amino)-7-cyclohexyl-5S-hydroxy-3-thia-heptansäure-methylester

6S-(4-Amino-piperidinocarbonyl-Pla-His-amino)-7-cyclohexyl-5S-hydroxy-3-thia-heptansäure-methylester

6S-(4-Amino-piperidinocarbonyl-Phe-β-Ala-amino)-7-cyclohexyl-5S-hydroxy-3-thia-heptansäure-methylester, F. 215-217°

6S-(4-Amino-piperidinocarbonyl-Phe-β-Ala-amino)-7-cyclohexyl-5S-hydroxy-3-thia-heptansäure-methylester -3,3-dioxid, TFA, F. 126-127°

7S-[4-Amino-piperidinocarbonyl-Phe-(S-Me-Cys)-amino]-8-cyclohexyl-6S-hydroxy-3-thia-octansäure-(N-isobutyl-amid), TFA, F. 148-149°

7S-[4-Amino-piperidinocarbonyl-Pla-(S-Me-Cys)-amino]-8-cyclohexyl-6S-hydroxy-3-thia-octansäure-(N-isobutyl-amid), TFA, F. 106-107°

7S-(4-Amino-piperidinocarbonyl-Phe-βAla-amino)-8-cyclohexyl-6S-hydroxy-3-thia-octansäure-(N-isobutyl-amid)

7S-(4-Amino-piperidinocarbonyl-Pla-βAla-amino)-8-cyclohexyl-6S-hydroxy-3-thia-octansäure-(N-isobutyl-amid), TFA, F. 130-131°

7S-(4-Amino-piperidinocarbonyl-Phe-His-amino)-8-cyclohexyl-6S-hydroxy-3-thia-octansäure-(N-isobutyl-amid)

7S-(4-Amino-piperidinocarbonyl-Pla-His-amino)-8-cyclohexyl-6S-hydroxy-3-thia-octansäure-(N-isobutyl-amid)

7S-[4-Amino-piperidinocarbonyl-Phe-(S-Me-Cys)-amino]-8-cyclohexyl-6S-hydroxy-3-thia-octansäure-methylester, TFA, F. 162-163°

7S-[4-Amino-piperidinocarbonyl-Pla-(S-Me-Cys)-amino]-8-cyclohexyl-6S-hydroxy-3-thia-octansäure-methylester, TFA, F. 88-89°

6S-[4-Amino-piperidinocarbonyl-Phe-(S-Me-Cys)-amino]-7-cyclohexyl-5S-hydroxy-3-thia-heptansäure-methylester-3,3-dioxid, TFA, F. 154-155°

6S-[4-Amino-piperidinocarbonyl-Pla-(S-Me-Cys)-amino]-7-cyclohexyl-5S-hydroxy-3-thia-heptansäure-methylester-3,3-dioxid, TFA, F. 161-162°

6S-(4-Amino-piperidinocarbonyl-Phe-His-amino)-7-cyclohexyl-5S-hydroxy-3-thia-heptansäure-methylester-3,3-dioxid

6S-(4-Amino-piperidinocarbonyl-Pla-His-amino)-7-cyclohexyl-5S-hydroxy-3-thia-heptansäure-methylester-3,3-dioxid

6S-(4-Amino-piperidinocarbonyl-Phe-Leu-amino)-7-cyclohexyl-5S-hydroxy-3-thia-heptansäure-methylester-3,3-dioxid

6S-(4-Amino-piperidinocarbonyl-Pla-Leu-amino)-7-cyclohexyl-5S-hydroxy-3-thia-heptansäure-methylester-3,3-dioxid

7S-[4-Amino-piperidinocarbonyl-Phe-(S-Me-Cys)-amino]-8-cyclohexyl-6S-hydroxy-3-thia-octansäure-methyl

ester-3,3-dioxid, TFA, F. 170-171°

7S-[4-Amino-piperidinocarbonyl-Pla-(S-Me-Cys)-amino]-8-cyclohexyl-6S-hydroxy-3-thia-octansäure-methyl-ester-3,3-dioxid, TFA, F. 130-131°

7S-(4-Amino-piperidinocarbonyl-Phe-His-amino)-8-cyclohexyl-6S-hydroxy-3-thia-octansäure-methylester-3,3-dioxid

7S-(4-Amino-piperidinocarbonyl-Pla-His-amino)-8-cyclohexyl-6S-hydroxy-3-thia-octansäure-methylester-3,3-dioxid

7S-(4-Amino-piperidinocarbonyl-Phe-Leu-amino)-8-cyclohexyl-6S-hydroxy-3-thia-octansäure-methylester-3,3-dioxid

7S-(4-Amino-piperidinocarbonyl-Pla-Leu-amino)-8-cyclohexyl-6S-hydroxy-3-thia-octansäure-methylester-3,3-dioxid

6S-[4-Amino-piperidinocarbonyl-Phe-(S-Me-Cys)-amino]-7-cyclohexyl-5R,S-hydroxy-3-thia-heptansäure-(N-isobutylamid)-3,3-dioxid

6S-[4-Amino-piperidinocarbonyl-Phe-(S-Me-Cys)-amino]-7-cyclohexyl-5S-hydroxy-3-thia-heptansäure-(N-isobutyl-amid)-3,3-dioxid, TFA, F. 176-177°

6S-[4-Amino-piperidinocarbonyl-Pla-(S-Me-Cys)-amino]-7-cyclohexyl-5S-hydroxy-3-thia-heptansäure-(N-isobutyl-amid)-3,3-dioxid, TFA, F. 130-131°

7-Cyclohexyl-5S-hydroxy-6S-H-Phe-(S-Me-Cys)-amino-3-thia-heptansäure-methylester-3,3-dioxid,  TFA,  F. 110°

6S-(4-Amino-piperidinocarbonyl-Phe-His-amino)-7-cyclohexyl-5S-hydroxy-3-thia-heptansäure-(N-isobutyl-amid)-3,3-dioxid

6S-(4-Amino-piperidinocarbonyl-Phe-Ala-amino)-7-cyclohexyl-5S-hydroxy-3-thia-heptansäure-(N-isobutyl-amid)-3,3-dioxid

6S-(4-Amino-piperidinocarbonyl-Phe-βAla-amino)-7-cyclohexyl-5S-hydroxy-3-thia-heptansäure-(N-isobutyl-amid)-3,3-dioxid

6S-(4-Amino-piperidinocarbonyl-Phe-Leu-amino)-7-cyclohexyl-5S-hydroxy-3-thia-heptansäure-(N-isobutyl-amid)-3,3-dioxid

6S-(4-Amino-piperidinocarbonyl-Phe-Met-amino)-7-cyclohexyl-5S-hydroxy-3-thia-heptansäure-(N-isobutyl-amid)-3,3-dioxid

6S-(4-Amino-piperidinocarbonyl-Phe-Nle-amino)-7-cyclohexyl-5S-hydroxy-3-thia-heptansäure-(N-isobutyl-amid)-3,3-dioxid    6S-(4-Amino-piperidinocarbonyl-Phe-Nva-amino)-7-cyclohexyl-5S-hydroxy-3-thia-heptansäure-(N-isobutyl-amid)-3,3-dioxid

6S-(4-Amino-piperidinocarbonyl-Phe-3-Pya-amino)-7-cyclohexyl-5S-hydroxy-3-thia-heptansäure-(N-isobutyl-amid)-3,3-dioxid

6S-(4-Amino-piperidinocarbonyl-Phe-Tia-amino)-7-cyclohexyl-5S-hydroxy-3-thia-heptansäure-(N-isobutyl-amid)-3,3-dioxid

6S-(4-Amino-piperidinocarbonyl-Phe-(2-Tiz)-amino]-7-cyclohexyl-5S-hydroxy-3-thia-heptansäure-(N-isobutyl-amid)-3,3-dioxid

7S-(4-Amino-piperidinocarbonyl-Phe-βAla-amino)-8-cyclohexyl-6S-hydroxy-3-thia-octansäure-methylester

7S-(4-Amino-piperidinocarbonyl-Pla-βAla-amino)-8-cyclohexyl-6S-hydroxy-3-thia-octansäure-methylester

7S-(4-Amino-piperidinocarbonyl-Phe-βAla-amino)-8-cyclohexyl-6S-hydroxy-3-thia-octansäure-methylester-3,3-dioxid

7S-(4-Amino-piperidinocarbonyl-Pla-βAla-amino)-8-cyclohexyl-6S-hydroxy-3-thia-octansäure-methylester-3,3-dioxid.

Beispiel 27

Ein Gemisch von 1 g 7-Cyclohexyl-5S-hydroxy-6S-[3-phenylpropionyl-(S-Me-Cys)-amino]-3-thia-heptansäure-methylester-3,3-dioxid, 50 ml Dioxan und 20 ml 2 n wässeriger NaOH-Lösung wird 3 Std. bei 20° gerührt. Nach üblicher Aufarbeitung erhält man 7-Cyclohexyl-5S-hydroxy-6S-[3-phenylpropionyl-(S-Me-Cys)-amino]-3-thia-heptansäure-3,3-dioxid, F. 106-107°

Analog erhält man durch Verseifung der entsprechenden Methylester:

7-Cyclohexyl-5S-hydroxy-6S-[P0A-(S-Me-Cys)-amino]-3-thia-heptansäure-3,3-dioxid, F. 101-102°.

6S-(4-BOC-amino-piperidinocarbonyl-Phe-His-amino)-7-cyclohexyl-5S-hydroxy-3-thia-heptansäure-3,3-dioxid

6S-(4-BOC-amino-piperidinocarbonyl-Pla-His-amino)-7-cyclohexyl-5S-hydroxy-3-thia-heptansäure-3,3-dioxid

6S-(4-BOC-amino-piperidinocarbonyl-Phe-Leu-amino)-7-cyclohexyl-5S-hydroxy-3-thia-heptansäure-3,3-dioxid

6S-(4-BOC-amino-piperidinocarbonyl-Pla-Leu-amino)-7-cyclohexyl-5S-hydroxy-3-thia-heptansäure-3,3-dioxid

6S-[4-BOC-amino-piperidinocarbonyl-Phe-(S-Me-Cys)-amino]-7-cyclohexyl-5S-hydroxy-3-thia-heptansäure-

18

3,3-dioxid

6S-[4-BOC-amino-piperidinocarbonyl-Pla-(S-Me-Cys)-amino]-7-cyclohexyl-5S-hydroxy-3-thia-heptansäure-3,3-dioxid

6S-(4-Amino-piperidinocarbonyl-Phe-His-amino)-7-cyclohexyl-5S-hydroxy-3-thia-heptansäure-3,3-dioxid

6S-(4-Amino-piperidinocarbonyl-Pla-His-amino)-7-cyclohexyl-5S-hydroxy-3-thia-heptansäure-3,3-dioxid

6S-(4-Amino-piperidinocarbonyl-Phe-Leu-amino)-7-cyclohexyl-5S-hydroxy-3-thia-heptansäure-3,3-dioxid

6S-(4-Amino-piperidinocarbonyl-Pla-Leu-amino)-7-cyclohexyl-5S-hydroxy-3-thia-heptansäure-3,3-dioxid

6S-[4-Amino-piperidinocarbonyl-Phe-(S-Me-Cys)-amino]-7-cyclohexyl-5S-hydroxy-3-thia-heptansäure-3,3-dioxid

6S-[4-Amino-piperidinocarbonyl-Pla-(S-Me-Cys)-amino]-7-cyclohexyl-5S-hydroxy-3-thia-heptansäure-3,3-dioxid

7S-(4-BOC-amino-piperidinocarbonyl-Phe-βAla-amino)-8-cyclohexyl-6S-hydroxy-3-thia-octansäure

7S-(4-BOC-amino-piperidinocarbonyl-Pla-βAla-amino)-8-cyclohexyl-6S-hydroxy-3-thia-octansäure

7S-[4-BOC-amino-piperidinocarbonyl-Phe-(S-Me-Cys)-amino]-8-cyclohexyl-6S-hydroxy-3-thia-octansäure

7S-[4-BOC-amino-piperidinocarbonyl-Pla-(S-Me-Cys)-amino]-8-cyclohexyl-6S-hydroxy-3-thia-octansäure, F. 71-72 °

7S-(4-BOC-amino-piperidinocarbonyl-Phe-βAla-amino)-8-cyclohexyl-6S-hydroxy-3-thia-octansäure-3,3-dioxid

7S-(4-BOC-amino-piperidinocarbonyl-Pla-βAla-amino)-8-cyclohexyl-6S-hydroxy-3-thia-octansäure-3,3-dioxid

7S-[4-BOC-amino-piperidinocarbonyl-Phe-(S-Me-Cys)-amino]-8-cyclohexyl-6S-hydroxy-3-thia-octansäure-3,3-dioxid

7S-[4-BOC-amino-piperidinocarbonyl-Pla-(S-Me-Cys)-amino]-8-cyclohexyl-6S-hydroxy-3-thia-octansäure-3,3-dioxid

7S-(4-Amino-piperidinocarbonyl-Phe-βAla-amino)-8-cyclohexyl-6S-hydroxy-3-thia-octansäure

7S-(4-Amino-piperidinocarbonyl-Pla-βAla-amino)-8-cyclohexyl-6S-hydroxy-3-thia-octansäure

7S-[4-Amino-piperidinocarbonyl-Phe-(S-Me-Cys)-amino]-8-cyclohexyl-6S-hydroxy-3-thia-octansäure

7S-[4-Amino-piperidinocarbonyl-Pla-(S-Me-Cys)-amino]-8-cyclohexyl-6S-hydroxy-3-thia-octansäure, TFA, F. 152-153°

7S-(4-Amino-piperidinocarbonyl-Phe-βAla-amino)-8-cyclohexyl-6S-hydroxy-3-thia-octansäure-3,3-dioxid

7S-(4-Amino-piperidinocarbonyl-Pla-βAla-amino)-8-cyclohexyl-6S-hydroxy-3-thia-octansäure-3,3-dioxid

7S-[4-Amino-piperidinocarbonyl-Phe-(S-Me-Cys)-amino]-8-cyclohexyl-6S-hydroxy-3-thia-octansäure-3,3-dioxid

7S-[4-Amino-piperidinocarbonyl-Pla-(S-Me-Cys)-amino]-8-cyclohexyl-6S-hydroxy-3-thia-octansäure-3,3-dioxid

8-Cyclohexyl-6S-hydroxy-7S-morpholinocarbonyl-Phe-βAla-amino-3-thia-octansäure

8-Cyclohexyl-6S-hydroxy-7S-morpholinocarbonyl-Pla-βAla-amino-3-thia-octansäure

8-Cyclohexyl-6S-hydroxy-7S-morpholinocarbonyl-Phe-(S-Me-Cys)-amino-3-thia-octansäure, F. 133-134°

8-Cyclohexyl-6S-hydroxy-7S-morpholinocarbonyl-Pla-(S-Me-Cys)-amino-3-thia-octansäure, F. 95-96 °

8-Cyclohexyl-6S-hydroxy-7S-morpholinocarbonyl-Phe-βAla-amino-3-thia-octansäure-3,3-dioxid

8-Cyclohexyl-6S-hydroxy-7S-morpholinocarbonyl-Pla-βAla-amino-3-thia-octansäure-3,3-dioxid

8-Cyclohexyl-6S-hydroxy-7S-morpholinocarbonyl-Phe-(S-Me-Cys)-amino-3-thia-octansäure-3,3-dioxid

8-Cyclohexyl-6S-hydroxy-7S-morpholinocarbonyl-Pla-(S-Me-Cys)-amino-3-thia-octansäure-3,3-dioxid

7S-[2-Benzyl-3-tert.-butylsulfonyl-propionyl-(S-Me-Cys)-amino]-8-cyclohexyl-6S-hydroxy-3-thia-octansäure, 2 Epimere, F. 136-137° bzw. 138-139°

7S-[2-Benzyl-3-tert.-butylsulfonyl-propionyl-(S-Me-Cys)-amino]-8-cyclohexyl-6S-hydroxy-3-thia-octansäure-3-oxid

7S-[2-Benzyl-3-tert.-butylsulfonyl-propionyl-(S-Me-Cys)-amino]-8-cyclohexyl-6S-hydroxy-3-thia-octansäure-3,3-dioxid.

Beispiel 28

Analog Beispiel 1 erhält man aus 6S-[4-CBZ-amino-piperidinocarbonyl-Phe-(S-Me-Cys)-amino]-7-cyclohexyl-5S-hydroxy-3-thia-heptansäure-(N-isobutylamid) das 6S-[4-Amino-piperidinocarbonyl-Phe-(S-Me-Cys)-amino]-7-cyclohexyl-5S-hydroxy-3-thia-heptansäure-(N-isobutylamid), TFA, F. 159-160°.

Beispiel 29

a) Analog Beispiel 6 erhält man aus 4-BOC-amino-piperidinocarbonyl-Phe-Leu-OH und 6S-Amino-7-cyclohexyl-5-oxo-3-thia-heptansäure-(N-isobutylamid)-3,3-dioxid das 6S-[4-BOC-amino-piperidinocarbonyl-Phe-Leu-amino]-7-cyclohexyl-5-oxo-3-thia-heptansäure-(N-isobutylamid)-3,3-dioxid.

b) Eine Lösung von 1 g des vorstehenden Ketoamids in 25 ml Methanol wird an 0,1 g 10%ig. Pd-C bei 20° und 1 bar bis zum Ende der $H_2$-Aufnahme hydriert. Nach Filtrieren und Eindampfen erhält man ein Gemisch von 5R- und 5S-Hydroxy-6S-(4-BOC-amino-piperidinocarbonyl-Phe-Leu-amino)-7-cyclohexyl-3-thia-heptansäure-(N-isobutyl- amid), das an Kieselgel getrennt werden kann.

Beispiel 30

Eine Lösung von 804 mg des nach Beispiel 29 a) erhältlichen Ketoamids und 1,43 g $Na_2CO_3$ . 10 $H_2O$ in 5 ml Methanol und 5 ml Wasser wird mit 70 mg Hydroxylaminhydrochlorid versetzt und 14 Std. bei 20° gerührt. Das ausgefallene Oxim wird abfiltriert, getrocknet, in 10 ml Methanol gelöst und an 0,4 g Raney-Ni bei 20° und 5 bar hydriert. Man filtriert den Katalysator ab, dampft das Filtrat ein und trennt das erhaltene Gemisch von 5R- und 5S-Amino-6S-[4-BOC-amino- piperidinocarbonyl-Phe-Leu-amino]-7-cyclohexyl-3- thia-heptansäure-(N-isobutylamid)-3,3-dioxid.

Beispiel 31

Eine Lösung von 1 mmol 6S-(4-BOC-amino-piperidinocarbonyl-Phe-βAla-amino)-7-cyclohexyl-5S-hydroxy-3-thia-heptansäure(N-isobutyl-amid) und 1 mmol 3-Chlorperbenzoesäure in 10 ml Chloroform wird 16 Std. bei 0° stehengelassen. Nach üblicher Aufarbeitung erhält man 6S-(4-BOC-amino-piperidinocarbonyl-Phe-βAla-amino)-7-cyclohexyl-5S-hydroxy-3-thia-heptansäure-(N-isobutyl-amid)-3-oxid.

Beispiel 32

Analog Beispiel 31, aber mit 2,5 mmol 3-Chlorperbenzoesäure erhält man 6S-(4-BOC-amino-piperidino-carbonyl-Phe-βAla-amino)-7-cyclohexyl-5S-hydroxy-3-thia-heptansäure-(N-isobutyl-amid)-3,3-dioxid.
Die nachfolgenden Beispiele betreffen pharmazeutische Zubereitungen.

Beispiel A: Tabletten

Ein Gemisch von 1 kg 6S-[4-Amino-piperidinocarbonyl-Phe-(S-Me-Cys)-amino]-7-cyclohexyl-5S-hydroxy-3-thia-heptansäure-(N-isobutylamid)-trifluoracetat, 4 kg Lactose, 1,2 kg Maisstärke, 200 g Talk und 100 g Magnesiumstearat wird in üblicher Weise zu Tabletten verpreßt, derart, daß jede Tablette 100 mg Wirkstoff enthält.

Beispiel B: Dragees

Analog Beispiel A werden Tabletten gepreßt, die anschließend in üblicher Weise mit einem Überzug aus Saccharose, Maisstärke, Talk, Tragant und farbstoff überzogen werden.

Beispiel C: Kapseln

500 g 6S-[4-Amino-piperidinocarbonyl-Phe-(S-Me-Cys)-amino]-7-cyclohexyl-5S-hydroxy-3-thia-heptansäure-(N-isobutylamid)-S,S-dioxid-trifluoracetat werden in üblicher Weise in Hartgelatinekapseln gefüllt, so daß jede Kapsel 500 mg Wirkstoff enthält.

Beispiel D: Injektionsgläser

Eine Lösung von 100 g 6S-(4-Amino-piperidinocarbonyl-Phe-Hisamino)-7-cyclohexyl-5S-hydroxy-3-thia-heptansäure- (N-isobutylamid)-bis-trifluoracetat in 4 l zweifach destilliertem Wasser wird mit 2 n Salzsäure auf pH 6,5 eingestellt, steril filtriert und in Injektionsgläser abgefüllt. Man lyophilisiert unter sterilen Bedingungen und verschließt steril. Jedes Injektionsglas enthält 50 mg Wirkstoff.

Beispiel E: Suppositorien

Man schmilzt ein Gemisch von 50 g 6S-[4-Amino-piperidinocarbonyl-Pla-(S-Me-Cys)-amino]-7-cyclohexyl-5S-hydroxy-3-thia-heptansäure-(N-isobutylamid)-trifluoracetat mit 10 g Sojalecithin und 140 g Kakaobutter, gießt in Formen und läßt erkalten. Jedes Suppositorium enthält 250 mg Wirkstoff.

**Patentansprüche**

1. Aminosäurederivate der Formel I

$$X-W-CR^1R^2-CO-Y-NH-CHR^4-CR^5-CH_2-(CR^6R^7)_rS(O)_t-CH_2CO-V-R^3 \qquad I$$

worin

X R$^8$, R$^8$-O-C$_m$H$_{2m}$-CO-, R$^8$-C$_m$H$_{2m}$-O-CO-, R$^8$-C$_m$H$_{2m}$-CO-, R$^8$-SO$_2$-, R$^9$R$^{10}$N-C$_m$H$_{2m}$-CO-, R$^{11}$-NH-C(=NH)-NH-C$_m$H$_{2m}$-CO-, R$^9$OOC-C$_m$H$_{2m}$-CO-, R$^9$O$_3$S-C$_m$H$_{2m}$-CO-, R$^9$-O- ( CH$_2$CH$_2$O )$_n$-C$_m$H$_{2m}$-CO-, oder A$_3$N$^+$-C$_m$H$_{2m}$-CO- An$^-$,

W O, NH, CH$_2$ oder S,

Y O oder 1 Aminosäurerest ausgewählt aus der Gruppe bestehend aus Abu, Ada, Ala, βAla, Arg, Asn, Asp, Bia, Cal, Cys, S-A-Cys, Dab, Gln, Glu, Gly, His, N(im) -A-His, Hph, Ile, Leu, tert.-Leu, Lys, Mal, Met, αNal, βNal, Nbg, Nle, Nva, Orn, Phe, Pia, Pro, Pya, Ser, Thr, Tia, Tic, Tiz, Trp, Tyr und Val,

V O oder NH,

R$^1$, R$^6$, R$^7$,

R$^9$ und R$^{10}$ jeweils H oder A,

R$^2$, R$^3$, R$^4$ und R$^8$ jeweils H, A, Ar, Ar-alkyl, Het, Het-alkyl, unsubstituiertes oder ein- oder mehrfach durch A, AO und/oder Hal substituiertes Cycloalkyl mit 3-7 C-Atomen, Cycloalkyl-alkyl mit 4-11 C-Atomen, Bicycloalkyl oder Tricycloalkyl mit jeweils 7-14 C-Atomen, Bicycloalkylalkyl oder Tricycloalkylalkyl mit jeweils 8-18 C-Atomen,

R$^5$ (H, OH), (H, NH$_2$) oder =O,

R$^9$R$^{10}$N auch eine unsubstituierte oder eine durch A, OH, NH$_2$, NHA, NA$_2$, NHAc, NH-CO-C$_x$H$_{2x}$-O-R$^{11}$, NH-CO-O-C$_x$H$_{2x}$-R$^{11}$, Hydroxy-alkyl, COOH, COOA, CONH$_2$, Amino-alkyl, HAN-alkyl, A$_2$N-alkyl, A$_3$N$^\oplus$alkyl An$^\ominus$, NH-CO-NH$_2$, NH-CO-NHA, Guanidinyl oder Guanidinyl-alkyl substituierte Pyrrolidino-, Piperidino-, Morpholino- oder Piperazino-gruppe,

R$^{11}$ A oder Ar-alkyl,

m und x jeweils 0, 1, 2, 3, 4, 5, 6, 7, 8, 9 oder 10,

n 1, 2, 3, 4 oder 5,

r 0, 1, 2 oder 3,

t 0, 1 oder 2,

Ar unsubstituiertes oder ein- oder mehrfach durch A, OA, Hal, CF$_3$, OH, NO$_2$, Hydroxy-alkyl, NH$_2$, NHA, NA$_2$, NHAc, NH-SO$_2$-A, SA, SO-A, SO$_2$-A, SO$_2$NH$_2$, SO$_2$NHA, COOH, COOA, CONH$_2$, CN, Amino-alkyl, HAN-alkyl, A$_2$N-alkyl, A$_3$N$^\oplus$-alkyl An$^\ominus$ und/oder Guanidinyl-alkyl substituiertes Phenyl oder unsubstituiertes Naphthyl,

Het einen gesättigten oder ungesättigten 5-oder 6-gliedrigen heterocyclischen Rest mit 1-4 N-, O- und/oder S-Atomen, der mit einem Benzolring kondensiert und/oder ein-oder mehrfach durch A, OA, Hal, CF$_3$, OH, NO$_2$, Carbonylsauerstoff, NH$_2$, NHA, NA$_2$, NHAc, NH-COOA, NHCOOAr, NHCOOCH$_2$Ar, NH-SO$_2$-A, SA, SO-A, SO$_2$-A, SO$_2$NH$_2$, SO$_2$NHA, COOH, COOA, CONH$_2$, CN, Ar, Ar-alkyl, Ar-alkenyl, Hydroxy-alkyl, Amino-alkyl, HAN-alkyl, A$_2$N-alkyl und/oder A$_3$N$^\oplus$-alkyl An$^\ominus$ substituiert sein kann und/oder dessen N- und/oder S-Heteroatome auch oxydiert sein können,

Hal F, Cl, Br oder J,

Ac A-CO-, Ar-CO-, Ar-alkyl-CO- oder A-NH-CO-,

An$^\ominus$ ein Anion, das auch fehlen kann, wenn stattdessen eine in der Verbindung der Formel I enthaltene Carboxygruppe in Form eines Carboxylatanions vorliegt,

-alkyl eine Alkylengruppe mit 1-8 C-Atomen und

A Alkyl mit 1-8 C-Atomen bedeuten,

worin ferner an Stelle einer oder mehrerer -CO-NH-Gruppen auch eine oder mehrere -CO-NA-Gruppen stehen können,

sowie deren Salze.

2.

a) 6S-(4-Amino-piperidinocarbonyl-Phe-(S-Me-Cys)-amino)-7-cyclohexyl-5S-hydroxy-3-thia-heptansäure-(N-isobutyl-amid) und dessen Salze;
b) 6S-(4-Amino-piperidinocarbonyl-Phe-(S-Me-Cys)-amino)-7-cyclohexyl-5S-hydroxy-3-thia-heptansäure-(N-isobutyl-amid)-S,S-dioxid und dessen Salze;
c) 7S-(4-Amino-piperidinocarbonyl-Phe-(S-Me-Cys)-amino)-8-cyclohexyl-6S-hydroxy-3-thia-octansäure-(N-isobutyl-amid) und dessen Salze;
d) 6S-(4-Amino-piperidinocarbonyl-Pla-(S-Me-Cys)-amino)-7-cyclohexyl-5S-hydroxy-3-thia-heptansäure-(N-isobutyl-amid) und dessen Salze;

e) 6S-(4-Amino-piperidinocarbonyl-Pla-(S-Me-Cys)-amino)-7-cyclohexyl-5S-hydroxy-3-thia-heptansäure-(N-isobutyl-amid)-S,S-dioxid und dessen Salze.

3. Verfahren zur Herstellung eines Aminosäurederivats der Formel I sowie von seinen Salzen, dadurch gekennzeichnet, daß man es aus einem seiner funktionellen Derivate durch Behandeln mit einem solvolysierenden oder hydrogenolysierenden Mittel in Freiheit setzt

oder daß man eine Carbonsäure der Formel II

$$X\text{-}G^1\text{-}OH \qquad II$$

worin $G^1$

(a)   fehlt,

(b)   $-W\text{-}CR^1R^2\text{-}CO\text{-}$,

(c)   $-W\text{-}CR^1R^2\text{-}CO\text{-}Y\text{-}$,

(d)   $-W\text{-}CR^1R^2\text{-}CO\text{-}Y\text{-}NH\text{-}CHR^4\text{-}CR^5\text{-}CH_2\text{-}(CR^6R^7)_r\text{-}S(O)_t\text{-}CH_2\text{-}CO\text{-}$ bedeutet,

oder eines ihrer reaktionsfähigen Derivate

mit einer Verbindung der Formel III

$$H\text{-}G^2\text{-}V\text{-}R^3 \qquad III$$

worin $G^2$

(a)   $-W'\text{-}CR^1R^2\text{-}CO\text{-}Y\text{-}NH\text{-}CHR^4\text{-}CR^5\text{-}CH_2(CR^6R^7)_r\text{-}S(O)_t\text{-}CH_2\text{-}CO\text{-}$,

(b)   $-Y\text{-}NH\text{-}CHR^4\text{-}CR^5\text{-}CH_2\text{-}(CR^6R^7)_r\text{-}S(O)_t\text{-}CH_2\text{-}CO\text{-}$ bedeutet,

(c)   $-NH\text{-}CHR^4\text{-}CR^5\text{-}CH_2\text{-}(CR^6R^7)_r\text{-}S(O)_t\text{-}CH_2\text{-}CO\text{-}$ bedeutet,

(d)   fehlt, und

W'   O, NH oder S bedeutet,

umsetzt,

und daß man gegebenenfalls in einer Verbindung der Formel I eine funktionell abgewandelte Amino- und/oder Hydroxygruppe durch behandeln mit solvolysierenden oder hydrogenolysierenden Mitteln in Freiheit setzt und/oder eine freie Aminogruppe durch behandeln mit einem acylierenden Mittel acyliert und/oder zur Herstellung einer Verbindung der Formel I, $R^5$ = (H, OH) oder (H, $NH_2$), ein Aminoketosäurederivat der Formel I, $R^5$ = 0, reduziert oder reduktiv aminiert und/oder eine Thioethergruppe zu einer Sulfoxid- oder Sulfongruppe oxydiert und/oder eine Verbindung der Formel I durch behandeln mit einer Säure in eines ihrer Salze überführt.

4. Verfahren zur Herstellung pharmazeutischer Zubereitungen, dadurch gekennzeichnet, daß man eine Verbindung der Formel I und/oder eines ihres physiologisch unbedenklichen Salze zusammen mit mindestens einem festen, flüssigen oder halbflüssigen Träger⁻ oder Hilfsstoff in eine geeignete Dosierungsform bringt.

5. Pharmazeutische Zubereitung, gekennzeichnet durch einen Gehalt an mindestens einer Verbindung der Formel I und/oder einem ihrer physiologisch unbedenklichen Salze.

6. Verwendung von Verbindungen der Formel I oder von deren physiologisch unbedenklichen Salzen zur Herstellung eines Arzneimittels.

7. Verwendung von Verbindungen der Formel I oder von deren physiologisch unbedenklichen Salzen bei der Bekämpfung der reninabhängigen Hypertension oder des Hyperaldosteronismus.